# EUROPÄISCHE PATENTSCHRIFT

(11) **EP 0 804 437 B1**
(45) Veröffentlichungstag und Bekanntmachung des Hinweises auf die Patenterteilung: **14.09.2005**
(21) Anmeldenummer: 95921800.9
(22) Anmeldetag: 31.05.1995
(51) Int. Cl.: C07D 487/04, C07D 513/04, A61K 31/41, A61K 31/505, A61K 31/535, A61K 31/55, A61K 31/54, C07D 498/04, C07F 9/6561

(54) **[a]-ANNELIERTE PYRROLDERIVATE UND DEREN ANWENDUNG IN DER PHARMAZIE**
[a]-ANNELATED PYRROLE DERIVATIVES AND PHARMACEUTICAL USES THEREOF
DERIVES DE PYRROLE a]-ANNELES ET LEUR UTILISATION DANS LE DOMAINE PHARMACEUTIQUE

(30) Priorität: 01.06.1994 DE 4419315
(43) Veröffentlichungstag der Anmeldung: 05.11.1997
(73) Patentinhaber: MERCKLE GMBH, 89143 Blaubeuren (DE)
(72) Erfinder: LAUFER, Stefan, D-89143 Blaubeuren (DE); STRIEGEL, Hans, Günther, D-89143 Blaubeuren (DE); DANNHARDT, Gerd, D-55127 Mainz (DE)
(74) Vertreter: Kinzebach, Werner
(86) Internationale Anmeldenummer: PCT/EP1995/002078
(87) Internationale Veröffentlichungsnummer: WO 1995/032971

(56) Entgegenhaltungen:
- EP-A- 0 397 175
- DE-A- 2 419 071
- US-A- 3 920 672
- US-A- 4 536 512
- JUSTUS LIEBIGS ANNALEN DER CHEMIE, Bd. 679, 1964 WEINHEIM DE, Seiten 139-144, T. PYL ET AL. 'Über bicyclisch Heterocyclen mit gemeinsamen Stickstoffatom'
- JOURNAL OF THE CHEMICAL SOCIETY, 1965 LETCHWORTH GB, Seiten 65-71, B.B. MOLLOY ET AL 'Studies of heterocyclic compounds'
- JOURNAL OF HETEROCYCLIC CHEMISTRY, Bd. 23, 1986 PROVO US, Seiten 1889-1892, C. GALERA ET AL. '2-Methylthiazolium salts as 1,4-dinucleophiles.'
- JOURNAL OF ORGANIC CHEMISTRY, Bd. 42, Nr. 14, 1977 EASTON US, Seiten 2448-2454, R. BUCHAN ET AL. 'Azaindolizines. 4. Synthesis and formylation of 8-azaindoles'
- CHEMICAL ABSTRACTS, vol. 76, no. 3, 1972 Columbus, Ohio, US; abstract no. 14391j, A.I. MEYERS ET AL. 'Dihydro-1,3-oxazines' Seite 374; & J. HETEROCYCL. CHEM. 1971, 8(5), 875-6,
- CHEMICAL ABSTRACTS, vol. 61, no. 1, 1964 Columbus, Ohio, US; V.K. KIBIREV ET AL. 'Heterocyclic analogs of aazulene' Spalte 5629g; & UKR. KHIM. ZH. 30(5), 488-95 (1964),
- CHEMICAL ABSTRACTS, vol. 64, no. 1, 1966 Columbus, Ohio, US; W. WEUFFEN ET AL. 'Relations of chemical constitution and bacteriostatic activity' Spalte 5488e; & PHARMAZIE 20(10), 629-33 (1965),
- CHEMICAL ABSTRACTS, vol. 84, no. 17, 1976 Columbus, Ohio, US; abstract no. 120952t, L. ALEKSEEVA 'Protonation of pyrrolo[1,2-a]imidazole and pyrrolo[1,2-a]benzimidazole derivatives' Seite 469; & SB. TR. VSES. N.-I. KHIM.-FARMATSEUVT. IN-TA 1974,(4)76-90,
- CHEMICAL ABSTRACTS, vol. 80, no. 25, 1974 Columbus, Ohio, US; abstract no. 146165f, A.A. DRUZHININA ET AL. '2,3-Dihydropyrrolo[1,2-a]imidazole' Seite 446; & SU,A,417 424 (ORDZHONIKIDZE) 28.Februar 1974
- CHEMICAL ABSTRACTS, vol. 68, no. 11, 1968 Columbus, Ohio, US; abstract no. 49510j, A. DRUZHININA ET AL. 'Imidazoles. XXXII. Pyrrolo[1,2-a]imidazoles' Seite 4791; & KHIM. GETEROTSIKL. SOEDIN 1967 (3), 532-5,
- CHEMICAL ABSTRACTS, vol. 77, no. 19, 1972 Columbus, Ohio, US; abstract no. 126481r, O. CEDER ET AL. 'Substitution reactions of pyrrolo[2,1-b]thiazoles' Seite 388; & TETRAHEDRON 1972,28 (16), 4341-52,
- CHEMICAL ABSTRACTS, vol. 77, no. 13, 1972 Columbus, Ohio, US; abstract no. 88394e, A.A. DRUZHININA ET AL. 'Imidazoles. LXXIV. Electrophilic substitutions in the pyrrolo[1,2-a]imidazole series' Seite 453; & KHIM. GETEROTSIKL. SOEDIN. 1972, (3), 405-9,
- CHEMICAL ABSTRACTS, vol. 77, no. 9, 1972 Columbus, Ohio, US; abstract no. 61153p, L.M. ALEKSEEVA ET AL. 'Protonation of pyrrolo[1,2-a]imidazole derivatives' Seite 432; & KHIM. GETEROTSIKL. SOEDIN. 1972, (4), 492 - 7,
- CHEMICAL ABSTRACTS, vol. 86, no. 17, 1977 Columbus, Ohio, US; abstract no. 121252t, A.A. DRUZHININA 'Imidazoles. XCI. Hydrolysis of 7-cyano derivatives of pyrrolo[1,2-a]imidazole' Seite 541; & KHIM. GETEROTSIKL. SOEDIN 1976, (12), 1658-9,
- CHEMICAL ABSTRACTS, vol. 106, no. 19, 1987 Columbus, Ohio, US; abstract no. 156332c, J.C. BRINDLEY ET AL. 'Routes to pyrrolo[2,1-b]thiazoles. Rotational isomerism of pyrrolo[2,1-b]thiazole-5-carbaldehydes' Seite 678; & J. CHEM. SOC., PERKIN TRANS 1 1986 (7), 1255-9,
- CHEMICAL ABSTRACTS, vol. 87, no. 1, 1977 Columbus, Ohio, US; abstract no. 5863q, A.A. DRUZHININA ET AL 'Studies on imidazoles' Seite 474; & KHIM. GETEROTSIKL. SOEDIN 1977, (2), 225-8,
- ARCHIV DER PHARMAZIE, Nr. 312, 1979, Seiten 896-907, WEINHEIM
- CHEMIKER ZEITUNG, Nr. 7/8, 1986, Seiten 867-871,
- ARCHIV DER PHARMAZIE, Nr. 321, 1988, Seiten 159-162, WEINHEIM

## Beschreibung

Die Erfindung betrifft Pyrrole, die an Bindung a anneliert sind und deren Anwendung in der Pharmazie sowie pharmazeutische Mittel, welche diese Verbindungen enthalten.

Es ist bekannt, daß die Metabolisierung von Arachidonsäure auf zwei verschiedenen Wegen erfolgt. Beim Cyclooxygenase-Weg wird unter Einwirkung des Enzyms Cyclooxygenase die Arachidonsäure zu Prostaglandinen metabolisiert. Beim Lipoxygenase-Weg wird die Arachidonsäure unter Einwirkung von Lipoxygenasen zu den sogenannten Leukotrienen metabolisiert.

Die Prostaglandine sind an der Entstehung von Entzündung, Fieber und Schmerz beteiligt, während die Leukotriene bei der Entstehung von Asthma, Entzündungen und Allergien eine wichtige Rolle spielen. Zur Bekämpfung dieser Symptome werden häufig nicht-steroidale Antiphlogistika, wie Arylessigsäure-, 2-Arylpropionsäure- und Anthranilsäure-Derivate, eingesetzt. Diese Derivate hemmen die Cyclooxygenase und verhindern somit die Bildung der Prostaglandine aus Arachidonsäure. Die Anwendung derartiger Derivate ist jedoch aufgrund ihrer Nebenwirkungen nicht unbedenklich. Arzneimittel, welche die Lipoxygenase hemmen, sind im Handel jedoch nicht erhältlich.

Die EP-A-397 175 beschreibt Pyrrolizinverbindungen der Formel: 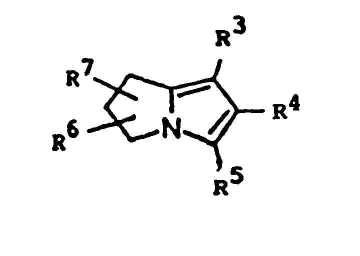 worin zwei der Reste R³, R⁴ und R⁵ unabhängig voneinander für H, C₅-C₈-Cycloalkyl, C₁-C₁₂-Alkyl oder Aryl, das gegebenenfalls durch ein oder zwei Reste substituiert ist, die ausgewählt sind unter Halogen, NO₂, C₁-C₄-Alkoxy, Hydroxy, C₁-C₄-Alkyl oder Phenoxy, stehen und der dritte der Reste R³, R⁴ und R⁵ für CHO, CO₂HO, COSC₁-C₄-Alkyl oder A - X steht, wobei A eine geradkettige oder verzweigte C₁-C₈-Alkylengruppe oder eine C₂-C₈-Alkenylengruppe bedeutet und X für CO₂H, SO₃H, CHO, OH oder SH steht. Diese Verbindungen sind Cyclooxygenase- und/oder Lipoxygenasehemmer und daher bei der Behandlung von Erkrankungen des rheumatischen Formenkreises und zur Prävention von allergisch induzierten Erkrankungen brauchbar.

Weitere Pyrrolizinverbindungen sind insbesondere beschrieben in US 3,920,672; DE 24 19 071 A; US 4,536,512; J. Liebigs Ann. Chem. 679, 139-144 (1964); J. Chem. Soc. 1965, 65-71; J. Heterocyclic Chem. 23, 1889-1892 (1986); J. Org. Chem. 42, 2448-2454 (1977),J. Heterocyclic Chem. 1971, 875-876.

Überraschenderweise wurde nun gefunden, daß bestimmte heterocyclischen Verbindungen den oben beschriebenen Pyrrolizinverbindungen in ihrer Wirkung und insbesondere in der Hemmung der Lipoxygenase überlegen sind.

Gegenstand der Erfindung sind daher heterocyclische Verbindungen der Formel I: 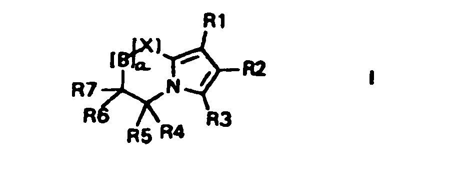 worin
zwei der Reste R¹, R² und R³, die gleich oder verschieden sein können, für einen Phenyl- oder Naphthylrest, der gegebenenfalls einen oder zwei Substituenten aufweist, die unter Halogen, Pseudohalogen, CF₃, NO₂, OH, C₁-C₈-Alkoxy, OCF₃, C₁-C₈-Alkyl oder Phenoxy ausgewählt sind, oder einen aromatischen heterocydischen Rest, der ausgewählt ist unter einem Pyrrol-, Imidazol-, Thiazol-, Thiadiazol-, Oxazol-, Isoxazol-, Pyridin- oder Chinolinrest und der gegebenenfalls durch Halogen, CF₃, C₁-C₈-Alkyl oder C₁-C₈-Alkoxy substituiert ist, stehen und
der dritte der Reste R¹, R² und R³ für H, CHO, CO₂H, COCO₂H, COCO₂ C₁-C₈-Alkyl oder A - Y steht,
- A: für C₁-C₈-Alkylen oder C₂-C₈-Alkenylen steht,
- Y: für CO₂H, SO₃H, OPO(OH)₂, PO(OH)₂ oder Tetrazolyl, CHO oder OH steht,
- R⁴, R⁵, R⁶ und R⁷,: die gleich oder verschieden sein können, für H oder C₁-C₈ -Alkyl stehen,
- X: für O, S, SO, SO₂ oder NR¹⁰ steht, wobei R¹⁰ für H, C₁-C₈-Alkyl oder Phenyl steht,
- B: für CR¹¹R¹² steht, wobei R¹¹ und R¹², die gleich oder verschieden sein können, für H oder C₁-C₈-Alkyl stehen und
- a: für 0, 1 oder 2 steht, und
deren optische Isomere, Salze und C₁-C₈-Alkyl-, Pivaloyloxymethyl-, Acetoxymethyl-, Phthalidyl-, Indanyl- und Methoxymethylester, ausgenommen eine Verbindung der Formel, worin R¹ für H, R² und R³ für Phenyl, a für O, X für NC₂H₅ und R⁴, R⁵, R⁶ und R⁷ für H stehen.

Die pharmazeutisch verträglichen Salze können im vorliegenden Fall Säureadditions- oder Basenadditionssalze sein. Für Säureadditionssalze verwendet man anorganische Säuren, wie Salzsäure, Schwefelsäure oder Phosphorsäure, oder organische Säuren, wie Weinsäure, Milchsäure, Citronensäure, Apfelsäure, Mandelsäure, Ascorbinsäure, Maleinsäure, Fumarsäure, Gluconsäure und dergleichen.

Zu Basenadditionssalzen zählen Salze der Verbindungen der Formel I mit anorganischen Basen, wie Natrium- oder Kaliumhydroxid oder mit organischen Basen, wie Mono-, Di- oder Triethanolamin.

Zu den Estern der Verbindungen der Formel I zählen insbesondere physiologisch leicht hydrolysierbare Ester, beispielsweise Alkyl-, Pivaloyloxymethyl-, Acetoxymethyl-, Phthalidyl-, Indanyl- und Methoxymethylester.

Der Ausdruck "Alkyl, Alkoxy etc." umfaßt geradkettige oder verzweigte Alkylgruppen, wie Methyl, Ethyl, n- und i-Propyl, n-, i- oder t-Butyl, n-Pentyl, Neopentyl, n-Hexyl etc.

Soweit nicht anders angegeben, steht "Alkyl" vorzugsweise für C₁-C₈-Alkyl, insbesondere für C₁-C₆-Alkyl und besonders bevorzugt für C₁-C₄-Alkyl.

Der Ausdruck "Halogenatom" umfaßt ein Fluor-, Chlor-, Brom- oder Jodatom und insbesondere ein Fluor- oder Chloratom. "Pseudohalogen" steht insbesondere für CN, OCN, SCN oder N₃.

"Alkylen" oder "Alkenylen" steht für geradkettige oder verzweigte Alkylen- oder Alkenylengruppen mit vorzugsweise 1 bis 6 bzw. 2 bis 6 und insbesondere 1 bis 4 bzw. 2 bis 4 Kohlenstoffatomen. Die Alkylengruppe und insbesondere die Methylengruppe ist bevorzugt.

"Acyl" steht für RCO, wobei R vorzugsweise die oben für "Alkyl" und "Aryl" angegebenen Bedeutungen besitzt. Acetyl ist besonders bevorzugt.

Bei dem "aromatischen heterocyclischen Rest" handelt es sich um einen 5- und 6-gliedrigen heterocyclischen Rest, einen Pyrrol-, Imidazol-, Thiazol-, Thiadiazol-, Oxazol-, Isoxazol-, Pyridin-, oder Chinolinrest. Wenn der Heterozyklus substituiert ist, sind 1, 2 oder 3 Substituenten vorhanden.

Die Substituenten der Phenyl- oder Naphthylgruppe sind vorzugsweise ausgewählt unter Halogen, insbesondere Fluor oder Chlor, CF₃, NO₂ und Phenoxy. Wenn es sich um einen Phenylring handelt, befinden sich die Substituenten vorzugsweise in m- und/oder p-Stellung.

Vorzugsweise steht der dritte der Reste R¹, R² und R³ in 5-Position des Pyrrolizidingerüstes.

Eine bevorzugte Ausführungsform sind die Verbindungen der obigen Formel I, worin zwei der Reste R¹, R² und R³ unabhängig voneinander für Phenyl, halogen- oder CF₃-substituiertes Phenyl (ein, zwei oder drei Halogenatome) oder einen 5- oder 6- gliedrigen heterocyclischen Ring der oben definierten Art stehen, und der dritte der Reste R¹, R² und R³ für A - Y steht, wobei A für C₁-C₈-Alkylen steht und Y für CO₂H, SO₃H, CHO oder COCO₂H steht.

Eine weiterhin bevorzugte Ausführungsform sind die Verbindungen der obigen Formel I, worin R¹ für Phenyl steht und R² für Phenyl oder halogen-substituiertes Phenyl steht:

Besonders bevorzugt steht A - Y für CH₂COOH.

Eine besonders bevorzugte Ausführungsform sind die Verbindungen der obigen Formel I, worin zwei der Reste R⁴ und R⁶ bzw. R⁵ und R⁷ zusammen für eine chemische Bindung stehen oder worin die Reste R⁴ - R⁷ für H oder Alkyl stehen. Diese Verbindungen besitzen die Formel:
Eine weitere bevorzugte Ausführungsform sind die Verbindungen der Formel I, worin zwei der Reste R¹, R² und R³ unabhängig voneinander für einen Phenylrest, einen durch ein oder zwei Halogenatome substituierten Phenylrest oder einen wie oben definierten aromatischen heterocyclischen Rest, der gegebenenfalls durch ein Halogenatom substituiert sein kann, stehen,
der dritte der Reste R¹, R² und R³ für ein Wasserstoffatom steht,
R⁴, R⁵, R⁶ und R⁷ die in Anspruch 1 angegebenen Bedeutungen besitzen;
X für S, SO oder SO₂ oder NR¹⁰ steht, wobei R¹⁰ für H, C₁-C₈-Alkyl oder Phenyl steht,
B für CH₂ steht und
a für 0, 1 oder 2 steht
oder worin einer oder zwei der Reste R¹, R² und R³ für einen wie oben definierten, gegebenenfalls substituierten aromatischen, heterocyclischen Rest stehen.

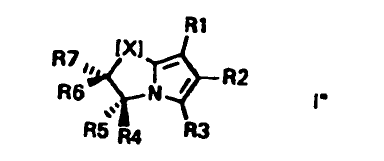

Die Reste R¹ bis R⁷ und X besitzen dabei die oben angegebenen Bedeutungen.
- X: steht vorzugsweise für S, SO und SO₂.
- B: steht vorzugsweise für CH₂ oder CH₂CH₂;
- a: steht vorzugsweise für 0 oder 1, insbesondere 0.

Soweit die erfindungsgemäßen Verbindungen Asymmetriezentren aufweisen, sind Racemate sowie optische Isomere (Enantiomere, Diastereomere) umfaßt.

Die Synthese der erfindungsgemäßen Verbindungen erfolgt analog zu den in den Schemata 1-5 beschriebenen Verfahren A bis O. Diese sind zum Teil in der EP-A-397 175 beschrieben; auf diese Publikation und die darin erwähnten Literaturzitate, wird hiermit Bezug genommen.

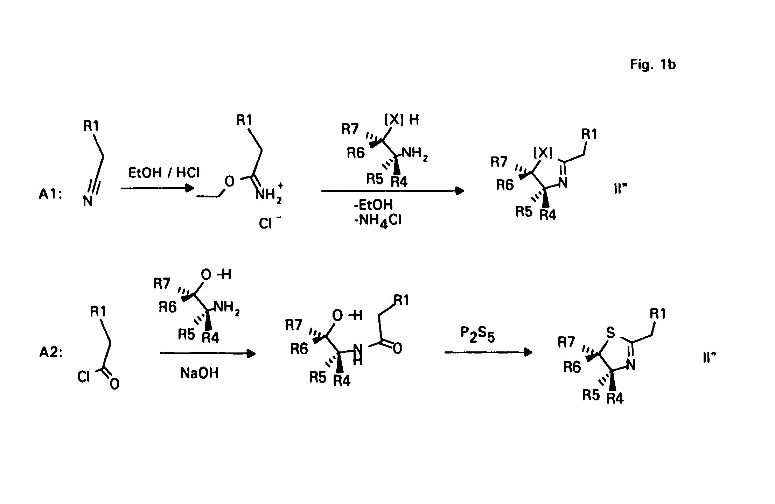

Ausgangsverbindungen für die Herstellung der erfindungsgemäßen Verbindungen sind die Verbindungen der Formel II: 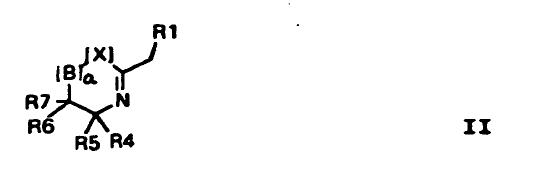 worin R¹, R⁴ bis R⁷, [B]ₐ und X die oben angegebenen Bedeutungen besitzen. Diese Verbindungen sind literaturbekannt oder sie werden analog zu bekannten Verfahren hergestellt, zum Beispiel durch Umsetzung der von D- und L-Aminosäuren abgeleiteten Aminoalkohole, von Aminothiolen und von Diaminen mit den Imidestern entsprechend substituierter Carbonsäuren (Fig. 1b A1/A2).

Die Verbindungen der Formel II werden mit den entsprechenden Verbindungen der Formel III 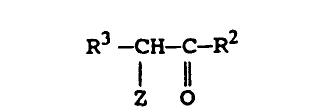 worin Z für Cl oder Br steht und R² und R³ die gewünschten Bedeutungen besitzen, umgesetzt. Die Verbindungen der Formel III sind ebenfalls literaturbekannt oder sie werden analog zu bekannten Verfahren hergestellt, beispielsweise werden diejenigen Verbindungen, bei denen R² für einen aromatischen heterocyclischen Rest steht, analog zu den von J.J. Riehl in C.R. Hebd. Seance. Acad. Sci. Ser.C (1957), 245, Seiten 1321-1322 beschriebenen Verfahren hergestellt.

Die Umsetzung nach Verfahren A erfolgt vorzugsweise in einem Ether oder aromatischen Kohlenwasserstoff, z.B. Diethylether, Benzol oder Toluol. Dabei fällt in der Regel das intermediar gebildete Quaternisierungsprodukt aus. Es wird unter Wasserausschluß isoliert und dann in einem inerten Lösungsmittel, z.B. Dichlormethan, mit Base, z.B. Triethylamin, behandelt. Die Reaktionstemperatur ist nicht kritisch, sie liegt im allgemeinen im Bereich von Raumtemperatur bis zur Siedetemperatur des Lösungsmittels.
Man erhält aus dieser Umsetzung die Verbindungen der Formel Ia: 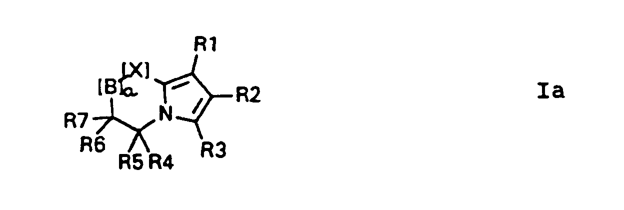 Wenn dabei mindestens einer der Reste R¹, R² und R³ für ein Wasserstoffatom steht, erhält man die Verbindungen der Formeln:

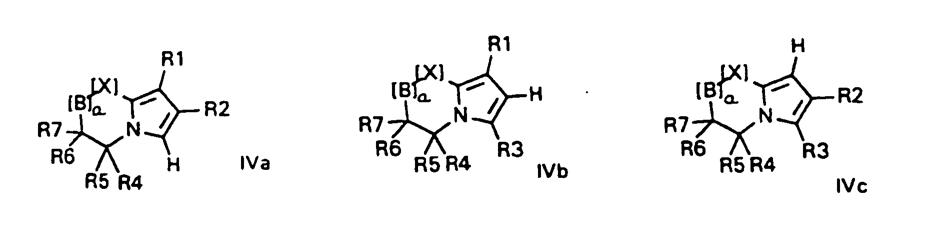

Je nach Stellung des Wasserstoffatoms leiten sich hiervon die Verbindungen der Reihe a, b oder c ab.

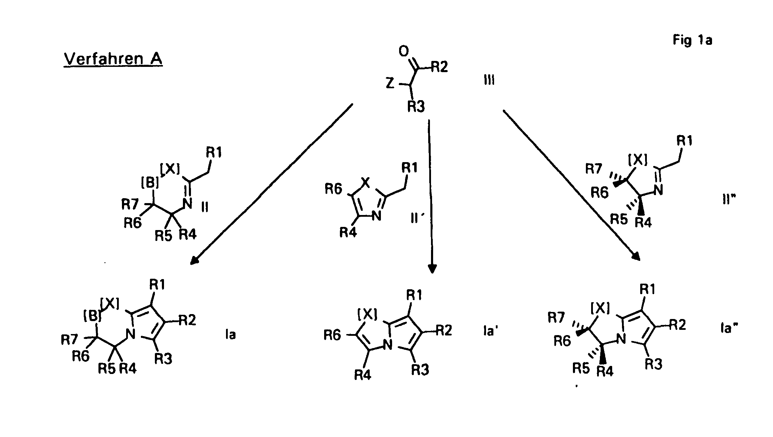

Diese Reaktion sowie die nachfolgend erwähnten Reaktionen sind in den Fig. 1a-c, 2, 3a, 3b, 4, 5a und 5b am Beispiel der Verbindungen der Reihe a skizziert. Analoges gilt für die Synthese und Derivatbildung der Verbindungen der Reihen b und c.

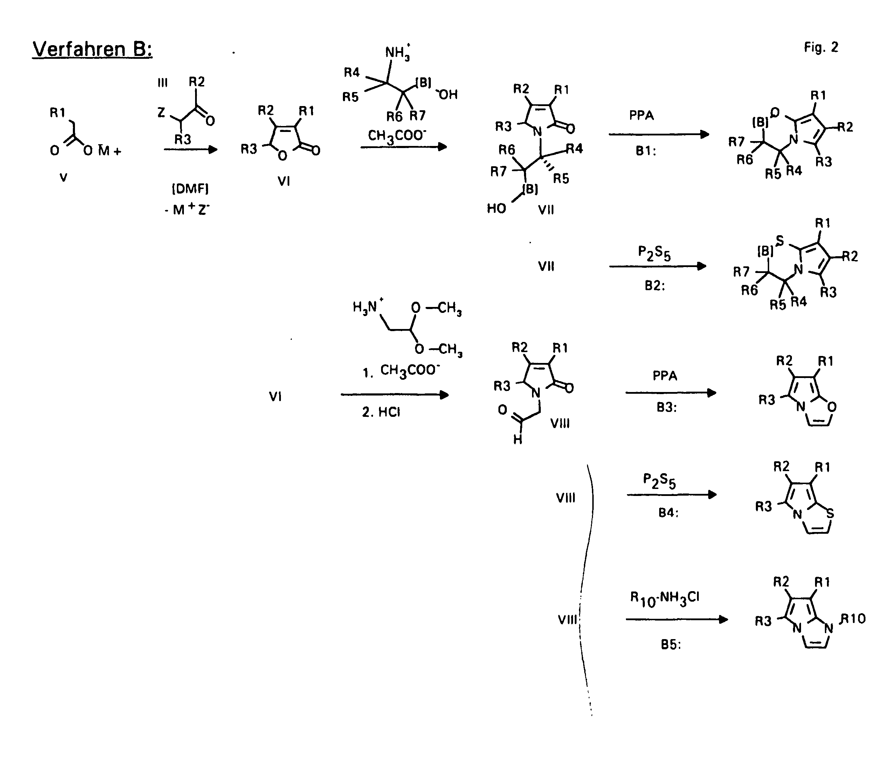

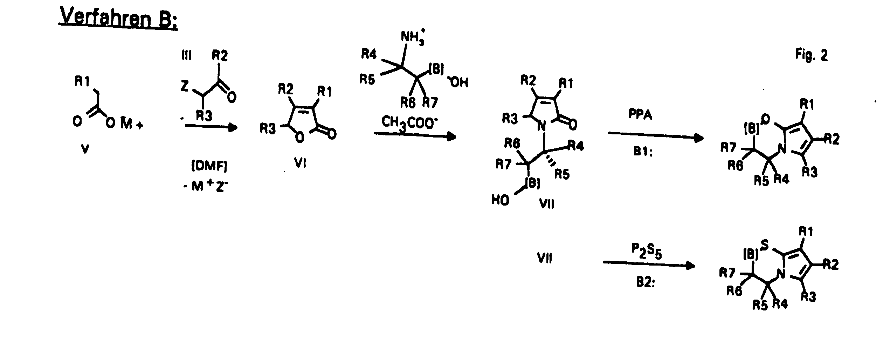

Zusätzlich zu dem in EP A-397 175 beschriebenen Verfahren (Verfahren A) findet zum Aufbau der Heterocyclen IVa, IVb und IVc ein weiteres Verfahren (Verfahren B) Anwendung (Fig. 2): Ausgangspunkt dieses Verfahrens sind entsprechend substituierte 2-(5H)Furanone (VI), die aus Carbonsäuresalzen der Struktur V und den Halogenaldehyden und -ketonen der Struktur III hergestellt werden (Fig. 2), analog zu den in der Literatur beschriebenen Methoden (a: Rio, G. und Sekiz, B. Bull. Soc. Chim. Fr. 1976, 1491, 1495. b: Padwa, A. , Brookhart, T., Dehm, D. und Wubbels, G., J. Am. Chem. Soc. 1978, 100, 8247, 8259). Analog zu literaturbekannten Methoden werden diese in 1,5-Dihydro-2-pyrrolone (VII bzw. VIII) umgewandelt (c: Matsuda et al. Yakugaku Zasshi 95,[1975] 190, 194 (C.A. 83 [1975] 42 780; d: Rio, G. und Sekiz, B., s.o.).

Die Cyclisierung zum annelierten Heterocyclus führt - abhängig vom verwendeten Kondensationsreagenz und von der zweiten funktionellen Gruppe des im vorhergehenden Schritt eingeführten bifunktionellen Amins NH₂-CR₄R₅CR₆R₇-[B]ₐ-OH bzw NH₂CH₂CH(OCH₃)₂ - (Formel I", Fig. 2: B1 /B2).

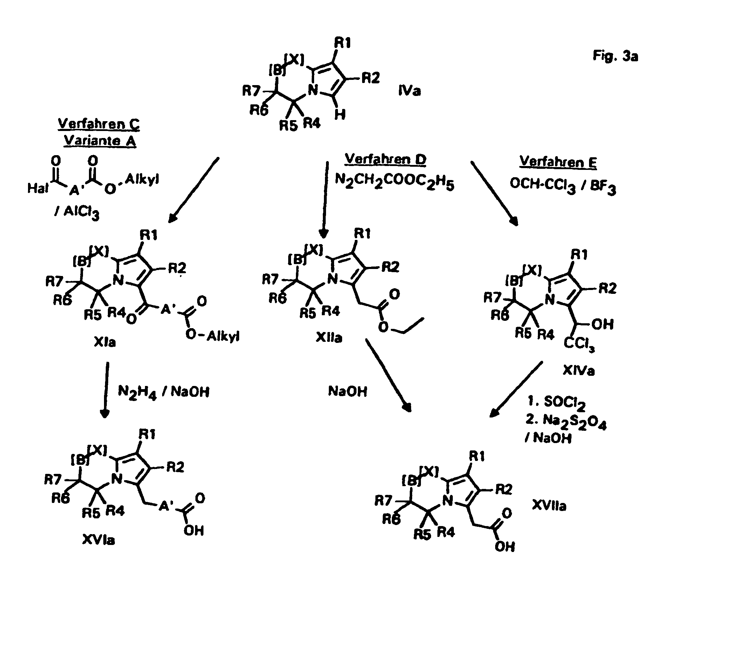

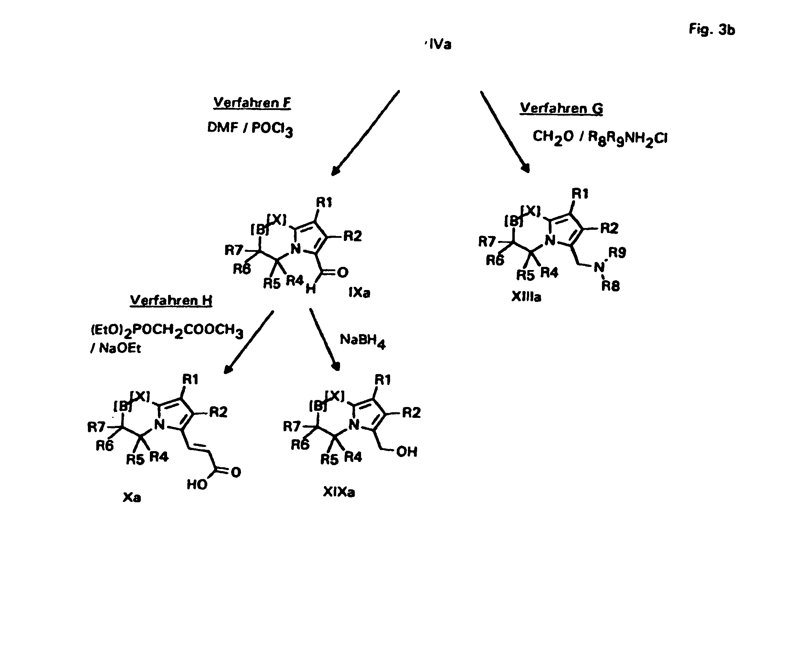

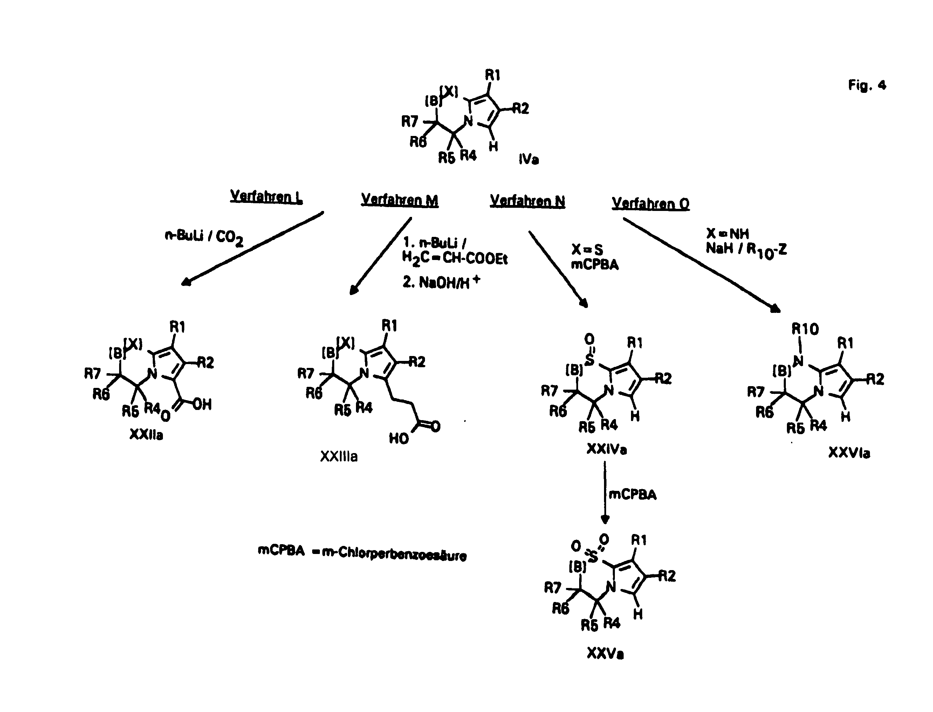

Gewünschtenfalls wird dann in das heterocyclische Grundgerüst ein weiterer Substituent nach Methoden eingeführt, die dem Fachmann bekannt sind. Zu diesen Methoden zählen beispielsweise:
a) Umsetzung einer Verbindung der Formel IV mit einem Carbonsäurehalogenid HalOC-A'-COOAlkyl, worin A' für eine chemische Bindung, C₁-C₇-Alkylen oder C₂-C₇-Alkenylen steht und Hal für Cl oder Br steht (Fig. 3a, Verfahren C/Variante A). Die erhaltene Verbindung der Formel Ia, worin einer der Reste R¹, R² und R³ für CO-A'-CO₂Alkyl steht, wird dann mit einem Reagens behandelt, das zur Reduktion der Carbonylgruppe zu einer CH₂-Gruppe geeignet ist, beispielsweise Hydrazin, NaCNBH₃ oder Zink-Amalgam. Die Umsetzung mit dem Carbonsäurehalogenid wird in einem inerten Lösungsmittel, z.B. Diethylether oder Tetrahydrofuran, gegebenenfalls in Gegenwart eines Katalysators durchgeführt. Die Reduktion mit Hydrazin erfolgt bevorzugt in einem hochsiedenden Alkohol, z.B. Diethylenglycol. Man erhält auf diese Weise die Verbindungen der Formel XVI.
b) Zur Einführung der besonders bevorzugten Gruppe CH₂CO₂H stehen mehrere Methoden zur Verfügung (siehe Fig. 3a, 3b, und 4). Die erste Möglichkeit besteht darin, daß man eine Verbindung der Formel IV mit Oxalylchlorid umsetzt (Fig. 5b), wobei man eine Verbindung der Formel I erhält, in der einer der Reste R¹, R² und R³ für COCO₂H steht. Diese Verbindung wird dann mit einem Reagens behandelt, das zur Reduktion der Ketocarbonylgruppe geeignet ist, beispielsweise Hydrazin, NaCNBH₃ oder Zink-Amalgam. Bevorzugt ist die Reduktion mit Hydrazin unter den Bedingungen einer Wolff-Kishner-Reduktion und insbesondere der Huang-Minlon-Variante davon (vgl. auch Punkt a) oben).
   Eine weitere Möglichkeit besteht darin, eine Verbindung der Formel IV mit einem Diazoessigsäurealkylester zu einer Verbindung der Formel I umzusetzen, in der einer der Reste R¹, R² und R³ für CH₂COOAlkyl steht. Diese Verbindung wird dann gewünschtenfalls einer Esterspaltung zu der entsprechenden freien Carbonsäure unterworfen. (Fig. 3a, XVII).
   Die Umsetzung mit dem Diazoessigester erfolgt in einem inerten Lösungsmittel, beispielsweise Toluol oder Xylol in Anwesenheit von Kupferpulver oder komplexen Kupfer-I-salzen oder Kupfer-II-salzen. Die Reaktion wird bei erhöhter Temperatur durchgeführt, zweckmäßigerweise bei der Siedetemperatur des verwendeten Lösungsmittels.
   Eine weitere Möglichkeit besteht in der Umsetzung einer Verbindung der Formel IV mit Chloral zu einer Verbindung der Formel XIV und Behandlung der aktivierten Verbindung mit einem Dithionit, beispielsweise Natriumdithionit oder einem Sulfinat, z.B. Hydroxymethansulfinsäure-Natriumsalz.
c) Die Einführung einer Formyl- oder Methylolgruppe in den Pyrrolring erfolgt durch Umsetzung einer Verbindung der Formel IV mit Phosphoroxychlorid/Dimethylformamid (siehe Fig. 3b). Die Umsetzung wird in einem inerten Lösungsmittel, beispielsweise Benzol, Toluol oder Xylol, bei erhöhter Temperatur, zweckmäßigervelse am Siedepunkt des verwendeten Lösungsmittels, durchgeführt. Man erhält eine Verbindung der Formel IX, worin einer der Reste R¹, R² und R³ für CHO steht. Diese Formylgruppe kann dann in üblicher Weise, beispielsweise mit Lithiumaluminiumhydrid in einem inerten Lösungsmittel, beispielsweise Tetrahydrofuran, oder mit Natriumborhydrid in wäßrig alkalischer Lösung zu der entsprechenden Hydroxymethylverbindung XIX reduziert werden (Fig. 3b).
   Weiter kann die Formylgruppe in einer unter üblichen Bedingungen durchgeführten Wittig-Reaktion in eine entsprechende Alkenylengruppe unter Bildung der Verbindung X überführt werden (siehe Verbindung X in Fig. 3b). Diese wiederum kann gewünschtenfalls in üblicher Weise zu der entsprechenden Alkylenverbindung (XXIII, Fig. 4) hydriert werden.
d) Umsetzung einer Verbindung der Formel IV mit einem Anhydrid der Formel: 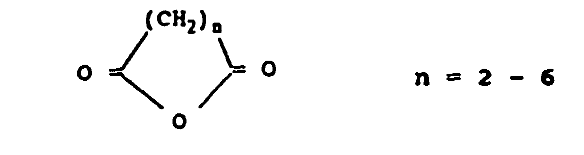 ergibt die entsprechenden Ketocarbonsäuren der Formel I, worin einer der Reste R¹, R² und R³ für CO(CH₂)ₐCO₂H steht. Die Ketocarbonylgruppe kann mit dem bereits erwähnten Reagens zu einer CH₂-Gruppe reduziert werden (siehe Fig. 3a, XI-XVI).
f) Eine Carboxylgruppe kann durch Umsetzung einer Verbindung der Formel IV mit n-Butyllithium in einem inerten Lösungsmittel bei tiefer Temperatur und anschließendes Durchleiten von CO₂-Gas durch die Lösung der gebildeten lithiumorganischen Verbindung eingeführt werden, man erhält Verbindungen der Formel XXII (siehe Verfahren L, Fig. 4).
g) Aus Carbonsäuren können durch Veresterung Ester und aus Estern Carbonsäuren durch Esterspaltung in üblicher Weise hergestellt werden.

Die Herstellung anderer erfindungsgemäßer Verbindungen erfolgt analog (Fig. 3a, 3b, 4), gegebenenfalls unter weiteren Umwandlungen, die dem Fachmann bekannt sind.

Die erfindungsgemäßen Verbindungen haben sich als potente Cyclooxygenase- und/oder Lipoxygenasehemmer erwiesen. Sie sind daher bei der Behandlung von Erkrankungen brauchbar, die mit einer Veränderung der Arachidonsäuremetabolisierung einhergehen. Insbesondere sind zu nennen Erkrankungen des rheumatischen Formenkreises und zur Prävention von allergisch induzierten Erkrankungen brauchbar. Die erfindungsgemäßen Verbindungen stellen somit wirksame Antiphlogistika, Analgetika, Antipyretika, Antiallergika und Broncholytika dar und sind antibronchokonstriktorisch wirksam und daher zur Thromboseprophylaxe und zur Prophylaxe des anaphylaktischen Schocks sowie zur Behandlung dermatologischer Erkrankungen, wie Psoriasis, Urtikaria, akute und chronische Exantheme allergischer und nicht-allergischer Genese brauchbar.

Die erfindungsgemäßen Verbindungen können entweder als einzelne therapeutische Wirkstoffe oder als Mischungen mit anderen therapeutischen Wirkstoffen verabreicht werden: Sie können als solche verabreicht werden, im allgemeinen werden sie jedoch in Form pharmazeutischer Mittel verabreicht, das heißt, als Mischungen der Wirkstoffe mit geeigneten pharmazeutischen Trägern oder Verdünnungsmitteln. Die Verbindungen oder Mittel können oral oder parenteral verabreicht werden, vorzugsweise werden sie jedoch in oralen Dosierungsformen gegeben.

Die Art des pharmazeutischen Mittels und des pharmazeutischen Trägers bzw. Verdünnungsmittels hängt von der gewünschten Verabreichungsart ab. Orale Mittel können beispielsweise als Tabletten oder Kapseln vorliegen und können übliche Exzipienzien enthalten, wie Bindemittel (z.B. Sirup, Akazia, Gelatine, Sorbit, Tragant oder Polyvinylpyrrolidon), Füllstoffe (z.B. Lactose, Zucker, Maisstärke, Calciumphosphat, Sorbit oder Glycin), Gleitmittel (z.B. Magnesiumstearat, Talcum, Polyethylenglykol oder Siliciumdioxid), disintegrierende Mittel (z.B. Stärke) oder Netzmittel (z.B. Natriumlaurylsulfat). Orale flüssige Präparate können in Form wäßriger oder öliger Suspensionen, Lösungen, Emulsionen, Sirupen, Elixieren oder Sprays usw. vorliegen oder können als Trockenpulver zur Rekonstitution mit Wasser oder einem anderen geeigneten Träger vorliegen. Derartige flüssige Präparate können übliche Additive, beispielsweise Suspendiermittel, Geschmacksstoffe, Verdünnungsmittel oder Emulgatoren, enthalten. Für die parenterale Verabreichung kann man Lösungen oder Suspensionen mit üblichen pharmazeutischen Trägern einsetzen.

Die erfindungsgemäßen Verbindungen oder Mittel können an ein Säugetier (Mensch und Tier) in Dosen von etwa 0,5 mg bis etwa 100 mg pro kg Körpergewicht pro Tag verabreicht werden. Sie können in einer Einzeldosis oder in mehreren Dosen verabreicht werden.

Die Wirksamkeit der erfindungsgemäßen Verbindungen läßt sich anhand der Hemmung der 5-Lipoxygenase oder der Cyclooxygenase bestimmen. Die Untersuchungen wurden nach Dannhardt et al.,J. Pharm. Pharmacol. 1992, 44:419-424 durchgeführt. Es wurde gefunden, daß die Verbindung des Beispiels 1 (X = N - Ph) die 5-Lipoxygenase bei 1 µmol/l zu 95 % hemmt, während die entsprechende Verbindung des Standes der Technik (X = CH₂) eine nur geringe Hemmwirkung zeigt.

Die nachfolgenden Beispiele erläutern die Erfindung. Alle Temperaturangaben sind unkorrigiert. Die IR-Spektren kristalliner Substanzen wurden, als KBr-Preßling aufgenommen, die öligen Substanzen als Film. Die NMR-Spektren sind, sofern nicht anders vermerkt, 200 MHz-Spektren, aufgenommen in CDCl₃ mit Tetramethylsilan (TMS) als internem Standard. Die IR-Spektren sind in cm⁻¹ und die NMR-Spektren in δ(ppm) angegeben.

### Beispiel 1

### 4,6,7-Triphenyl-2,3-dihydro-Pyrrolo-[2,1-b]-imidazol-essigsäure

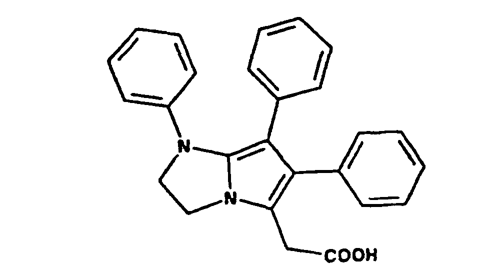

### A) 2-Benzyl-N-Phenylimidazolin

0,88 Mol HCl-Gas wurden bei -20°C in 65 ml absolutem Ethanol gelöst. Unter Kühlung wurden dann 0,8 Mol Benzylcyanid so zugetropft, daß die Innentemperatur 15°C nicht überschritten hat. Nach 2-stündigem Rühren bei 15 - 20°C wurde eine Lösung von 0,76 Mol Ammoniak-Gas in 160 ml Ethanol zugetropft, wobei darauf geachtet wurde, daß die Temperatur 20°C nicht überschritt. Anschließend tropfte 0,8 Mol N-Phenylethylendiamin zu. Nach einstündigem Rühren bei Raumtemperatur wurde das Ethanol bis zu einer Sumpftemperatur von 110°C abdestilliert. Nach dem Abkühlen wurde mit 250 ml Wasser versetzt, mit NaOH alkalisch gemacht und mehrfach mit Methylenchlorid extrahiert. Der nach dem Verdampfen der getrockneten organischen Phasen verbleibende Rückstand wurde fraktioniert destilliert. Das gewünschte Produkt geht bei 135 - 145°C/0,5 mbar über. Ausbeute 35 %.
- IR-Spektrum:: 3270, 3055, 2930, 1621, 1613, 1490, 1385, 1312, 1255, 1178, 1025, 754, 734, 715 cm⁻¹
- ¹H-NMR-Spektrum (200 MHz):: 7,3-6,9 (m, 10H, arom.), 3,86-3,77 (m, 4H,(N-CH₂-CH₂-N), 3,63(s,2H, -CH₂-Ph)

### 4,6,7-Triphenyl-2,3-dihydro-pyrrolo-[2,1-b]-imidazol

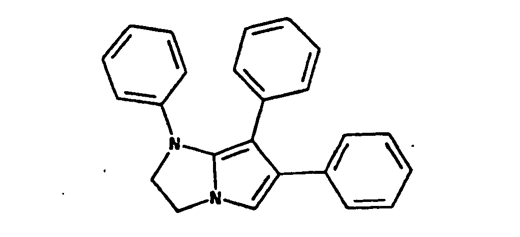

Zu einer Lösung von 48 mmol Phenacylbromid in 60 ml trockenem Diethylether wurden 48 mmol 2-Benzyl-1-phenyl-Δ-2-imidazolin, das in 60 ml Diethylether gelöst war, unter Rühren getropft. Nach beendeter Zugabe wurde eine weitere Stunde gerührt und dann über Nacht stehen gelassen. Die Suspension wurde vorsichtig dekantiert und der Niederschlag wurde in 250 ml trockenem Methylenchlorid aufgenommen und mit 160 ml Diethylether versetzt. Anschließend tropfte man 48 ml trockenes Triethylamin unter Rühren zu. Nach beendeter Zugabe wurde weitere 6 Stunden bei Raumtemperatur gerührt. Die flüchtigen Bestandteile wurden unter vermindertem Druck verdampft und der verbleibende Rückstand wurde säulenchromatographisch gereinigt (Kieselgel, Laufmittel THF). Das ölige Produkt wurde mit Diethylether zur Kristallisation gebracht. Ausbeute: 48 %.
Schmelzpunkt: 172°C.
- IR-Spektrum:: 1596, 1555, 1493, 1430, 1393, 1315, 1100, 1019, 757, 697,
- 1H-NMR-Spektrum (200 MHz): 7,48 - 6,67 (m, 15H, arom), 6,59 (s, 1H, -CH=C), 4,45 (t, 2H, J=10 Hz), 4,19 (t, 2H, J = 10 Hz)

### C) 4,6,7-Triphenyl-2,3-dihydro-pyrrolo-[2,1-b]-imidazol)-5-yl-essigsäureethylester

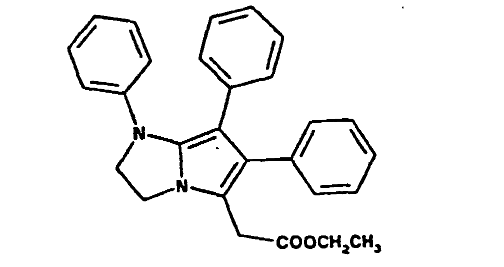

10 mmol des Produktes aus Stufe B wurden in 80 ml trockenem Toluol gelöst und mit 0,6 g Kupferpulver und 40 mmol Diazoessigester versetzt. Die Mischung wurde 1 Stunde unter Rückfluß erhitzt. Nach Abfiltrieren des Kupferpulvers und Abziehen des Lösungsmittels wurde der Rückstand säulenchromatographisch gereinigt (neutrales Aluminiumoxid, Laufmittel n-Hexan/Isopropylether). Das Produkt wurde aus Isopropanol umkristallisiert.
- Ausbeute:: 31 %
- Schmelzpunkt:: 116°C
- IR-Spektrum:: 1723, 1598, 1559, 1495, 1435, 1190, 1026, 748, 694
- ¹H-NMR-Spektrum:: 7,31-6,54(m, 15H, arom); 4,43 (t,2H,J=7,3 Hz); 4,19 (t, 2H, J = 7,3), 4,20 (q, 2H, J = 7,2, ethyl); 3,55 (s, 2H, -CH₂-C=O); 1,30 (t,3H, J = 7,2 ethyl)

### D) 4,6,7-Triphenyl-2,3-dihydro-pyrrolo[2,1-b]-imidazol-5-ylessigsäure

Die Esterverbindung aus Stufe C wurde in Methanol gelöst und eine Stunde mit einem 10%igen molaren Überschuß an fein gepulvertem Natriumcarbonat gerührt. Das nach dem Abfiltrieren der Feststoffe erhaltene Filtrat wurde verdampft und der Rückstand wurde mit Isopropylether gewaschen, wobei 89 % der Titelverbindung in Form des Natriumsalzes erhalten wurden, aus dem gewünschtenfalls durch Ansäuern die freie Säure erhalten werden kann. Schmelzpunkt: 181°C.
- IR-Spektrum:: 1704 (C = 0)
- ¹H-NMR-Spektrum:: 7,30-6,48(m,15H, Ar.); 4,42 (t,2H, J = 7,3 Hz); 4,21 (t,2H, J = 7,3 Hz); 3,48 (s, 2H, CH₂)

### Beispiel 2 (Referenz beispiel)

### {[3-Methyl-6-(4-chlorphenyl)-7-phenyl]pyrrolo-[2,1-b]-thiazol-5-yl}essigsäure

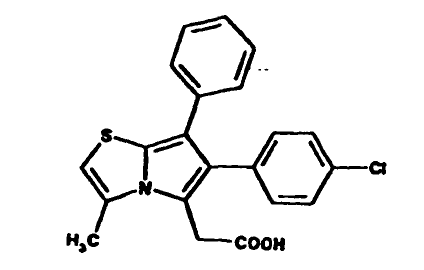

### A) 2-Benzyl-4-methylthiazol

15 g Thiophenylacetamid (0,1 Mol) wurden in 200 ml Toluol suspendiert und die Toluolsuspension wurde zum Rückfluß erhitzt. Zu der gebildeten Lösung wurden 13,9 g Chloraceton (0,15 Mol) getropft und anschließend wurde das Gemisch eine weitere Stunde unter Rückfluß erhitzt. Nach dem Abkühlen im Eisbad wurde der Niederschlag abdekantiert. Das Thiazol wurde mit Natriumdicarbonatlösung aus dem Hydrochlorid freigesetzt und in Isopropylether aufgenommen. Die wäßrige Lösung wurde mehrmals mit Isopropylether extrahiert. Die Isopropyletherphase wurde über Natriumsulfat getrocknet und verdampft und der ölige Rückstand wurde fraktioniert destilliert. Man erhielt 14 g (74 % der Titelverbindung). Für die weitere Umsetzung kann man den nach Verdampfen des Isopropylethers erhaltenen Rückstand direkt ohne Destillation verwenden.
- ¹H-NMR-Spektrum:: 2,426 (d; J= 0,8 Hz,3H); 4,29(s;2H) 6,725 (q, J=0,8Hz,1H) 7,0 - 7,5 (5H, m)

### B) 6-(4-Chlorphenyl)-3-methyl-7-phenyl-pyrrolo-[2,1-b]thiazol

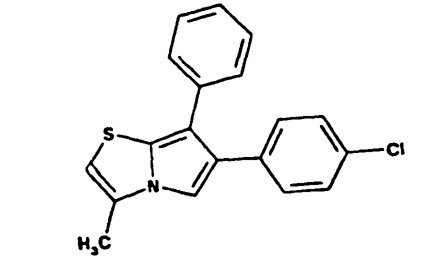

(1) 9,4 g 2-Benzyl-4-methylthiazol (0,05 Mol) und 11,6 g w-Brom-4-chloracetophenon (0,05 Mol) wurden 2 Stunden im Wasserbad auf 80°C erhitzt, wobei sich nach anfänglicher Verflüssigung der Mischung wieder ein Feststoff bildete. Die erstarrte Masse wurde zerkleinert und mit Chloroform ausgerührt. Der kristalline Feststoff wurde abgesaugt, mit Chloroform gewaschen und anschließend getrocknet. Ausbeute: 13,6 g (66 %)
(2) 13 g des gemäß (1) erhaltenen Thiazoliumsalzes wurden mit 3,8 g Triethylamin in 200 ml Chloroform 2 Stunden unter Rückfluß erhitzt. Nach dem Abkühlen wurde abfiltriert und das Filtrat wurde 2 mal mit 200 ml Wasser extrahiert. Die Chloroformphase wurde anschließend getrocknet und verdampft. Man erhielt 8,2 g (67 %) der Titelverbindung als zähe Masse.
   - ¹H-NMR-Spektrum:: 2,395(d,3H, J=0,8Hz); 6,308(q,1H, J=0,8Hz); 7,16 (s,1H); 7,1-7,4 (m; 9H; Ar2)

### C) 2-[6-(4-Chlorphenyl)-3-methyl-7-phenyl-pyrrolo-[2,1-b]-thiazol-5-yl]-2-oxo-essigsäure-ethylester

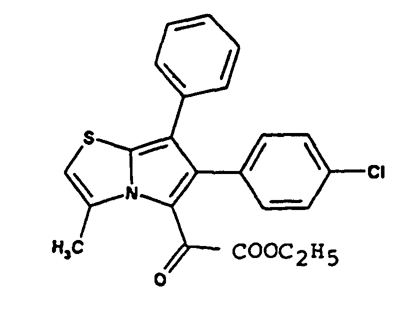

0,75 g des gemäß (B) erhaltenen Produktes (2,3 mmol) wurden in 10 ml Methylenchlorid gelöst und anschließend wurden zu dieser Lösung bei Raumtemperatur 0,31 g Oxalsäureethylestersäurechlorid in 2 ml Methylenchlorid getropft. Anschließend wurde die Mischung noch eine Stunde gerührt, mit 5 ml Wasser versetzt und die Methylenchloridphase wurde dann abgetrennt, über Natriumsulfat getrocknet und im Vakuum verdampft. Man erhielt 0,6 g der Titelverbindung mit einem Schmelzpunkt von >200 °C (Zersetzung).
- IR-Spektrum:: 1725, 1711
- ¹H-NMR-Spektrum:: 1,1(t,3H, J = 7 Hz, CH₃); 2,71(d,3H,J=0,8Hz); 3,72(q,2H,CH₂,J=7Hz);6,3(q,1H, J=0,8Hz); 7,07 7,41(m,9H,Ar.)

### D) 2-[6-(4-Chlorphenyl)-3-methyl-7-phenyl-pyrrolo--[2,1-b]-thiazol-5-yl]-essigsäure

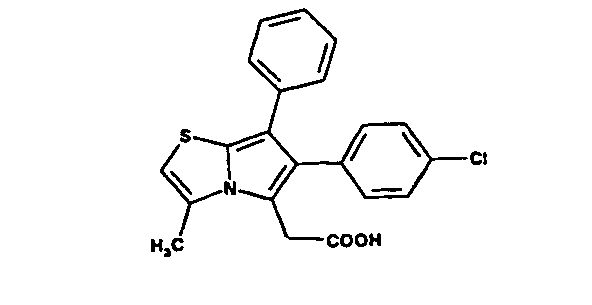

Die Titelverbindung wurde nach der allgemeinen Arbeitsvorschrift in der EP-A-397 175 zur Herstellung der 4-(Diphenyl-2,3-dihydro-1H-pyrolizinyl)buttersäuren aus den entsprechenden 4-oxobuttersäuren durch Reduktion mit Hydrazin hergestellt. Man erhielt die Titelverbindung in einer Ausbeute von 73 % und mit einem Schmelzpunkt von 194 - 196°C (Zersetzung).
- IR-Spektrum:: 3430, 1710, 1595, 1518, 1396, 1222, 1088, 1011, 841, 711 cm⁻¹
- ¹H-NMR-Spektrum:: 7,3-7,03(m,9H,arom.); 6,325-6,320(d,1H,J=0,8Hz);3,76(s,-CH₂-,2H); 2,569-2,566(d,2H,J=0,8Hz)

### Beispiel 3 (Referenzbeispiel)

### {[6-(4-Chlorphenyl)-3-methyl]-pyrrolo-[2,1-b) thiazol-5yl}-essigsäure

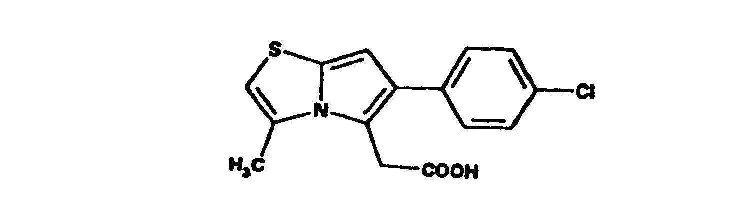

### A) 3-Methyl-6-(4-chlorphenyl)-pyrrolo-[2,1-b]-thiazol

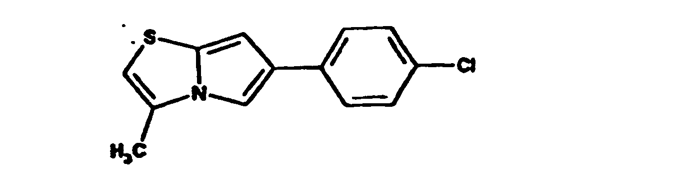

Die Herstellung erfolgte analog der Stufe (B) des Beispiels 2. Man erhielt die Titelverbindung in einer Ausbeute von 53 % und mit einem Schmelzpunkt von 116°C.
- ¹H-NMR-Spektrum:: 2,365(3H,d,J=0,8Hz, CH₃), 6,262 (q,1H,0,8 Hz) 6,480(d,1H,J=1,6Hz), 7,289 (d, 1H, J=1,6 Hz) 7,3-7,6(AA'BB',4H)

### B) 6-(4-Chlorphenyl)-3-methyl-pyrrolo-[2,1-b]-thiazol-5-ylessigsäure-ethylester-3-methyl

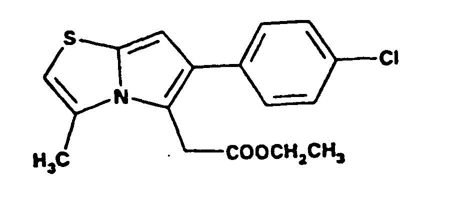

1,2 g der gemäß Stufe (A) erhaltenen Verbindung (0,005 Mol) wurden in 20 ml Toluol in der Hitze gelöst und mit 0,5 g Kupferpulver versetzt. 2,3 g (20 mmol) Diazoessigester wurden in kleinen Anteilen zugegeben und das Reaktionsgemisch wurde weitere 8 Stunden unter Rückfluß erhitzt. Das Kupferpulver wurde abfiltriert, das Filtrat zur Trockene verdampft und der Rückstand säulenchromatographisch gereinigt. Man erhielt 0,8 g der Titelverbindung als Öl.
- IR-Spektrum:: 1728 (C=O)
- ¹H-NMR-Spektrum:: 1,12(t,3H,CH₃, J=6,7 Hz); 2,62(d,3H, J=0,8Hz, CH₃); 3,58(q,2H,CH₂,J=6,7), 6,35(s,1H); 6,58 (q,1H,J=0,8Hz); 7,18-7,41(AA'BB',Ar.)

### C) 6-(4-Chlorphenyl)-3-methyl-pyrrolo-[2,1-b]-thiazol-5-yl-2-oxo-essigsäureethylester:

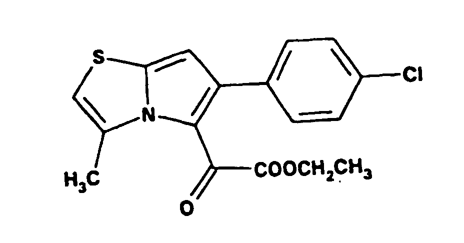

Die Synthese erfolgte analog der Stufe (C) des Beispiels 2. Man erhielt 67 % der Titelverbindung mit einem Schmelzpunkt > 200°C (Zersetzung).
- IR-Spektrum:: 1732, 1617
- ¹H-NMR-Spektrum:: 1,087(t,3H, CH₃) 2,698(d,3H,J=0,8Hz), 3,664(q,2H,CH₂) 6,37(1H,s), J =6,7 Hz, 6,523 (q,1H, J=0,8Hz); 7,20-7,40(AA'BB',4H).

### D) {[6-(4-Chlorphenyl)-3-methyl-pyrrolo-[2,1-b]thiazol-5yl}-essigsäure

Die Synthese erfolgte analog der Stufe (D) des Beispiels 2. Man erhielt 81 % der Titelverbindung mit einem Schmelzpunkt >190°C (Zersetzung).
- IR-Spektrum:: 1690 cm⁻¹ (C = O)
- ¹H-NMR-Spektrum:: 2,52(d, 3H, CH₃); 4,00 (s, 2H, CH₂); 6,15 q, (q, 1H,=CH),6,21 (s, 1H,=CH) 7,25-7,5(m,4H,Ar).

### Beispiel 4 (Referenzbeispiel)

### 2-[6-(4-Chlorphenyl)-2,3-dimethyl-7-phenyl-pyrrolo[2,1-b]thiazo 1-5-yl]-essigsäure

### nach Verfahren A3:

### A) 2-Benzyl-4,5-dimethyl-thiazol

### analog Beispiel 2, Buchstabe A:

Zur heißen Lösung von Phenylthioacetamid (3,75 g, 0.025 Mol) in Toluol (40 mL) wird 3-Brom-2-butanon getropft und weitere 2 Std refluxiert. Der nach Abkühlen vom Toluol dekantierte Rückstand wird in Chloroform (50 mL) gelöst und mit Na₂CO₃-Lsg (20 mL, 10%) neutralisiert, die Chloroformphase getrocknet (Na₂SO₄), im Vakuum eingeengt und der verbleibende braungelbe Rückstand destilliert: gelbliches Öl, Sdp 98-100 °C (5·10⁻¹).
C₁₂H₁₃NS (MG=203.3)
IR (cm⁻¹): 2915, 1599, 1551, 1488, 1449, 1424, 1122, 1026, 755, 702.
¹H-NMR (200MHz, CDCl₃): 7.40-7.10 (m, 5H, arom.), 4.208 (s, 2H, CH₂), 2.294 (s, 3H, CH₃), 2.258 (s, 3H, CH₃).

### B) 2-Benzyl-3-[2-(4-chlorphenyl)-2-oxo-ethyl]-4,5-dimethylthiazol iumbromid

C₂₀H₁₉BrClNOS (MG=436.8)
zähe Masse aus CHCl₃, Substanz nicht isoliert sondern als
Rohprodukt weiter umgesetzt.

### C) 6-(4-Chlorphenyl)-2,3-dimethyl-7-phenyl-pyrrolo[2,1-b]thiazol

C₂₀H₁₆ClNS (MG=337.87)
IR (cm ⁻¹): 1594, 1514, 1411, 1088, 831, 756, 696.
¹H-NMR (200MHz, CDCl₃): 7.35-7.10 (m, 5H, arom.), 7.07(s, 1H, pyrr.), 2.31 (s, 3H, CH₃), 2.29 (s, 3H, CH₃).

### D) 2-[6-(4-Chlorphenyl)-2,3-dimethyl-7-phenyl-pyrrolo[2,1-b]thiazo 1-5yl]-2-oxo-essigsäureethylester

C₂₄H₂₀ClNO₃S (437.9)
Schmp.: 167 °C
IR (cm⁻¹): 1724, 1623, 1396, 1274, 1244, 1020, 750, 694. ¹H-NMR (200MHz, CDCl₃): 7.40-7.05 (m,9H,2 arom.), 3.635 (q,2H,J=7.1 Hz, CH₂), 2.564 (q, 3H, J=0.6Hz, CH₃), 2.393 (q,3H,J=0.6Hz, CH₃);', 1.10 (t, J=7.1Hz, CH₃).

### E) 2-[6-(4-Chlorphenyl)-2,3-dimethyl-7-phenyl-pyrrolo[2,1-b]thia zol-5-yl)-essigsäure

C₂₂H₁₈ClNO₂S (MG=395.9)
Schmp.: 166 **°** C
IR (cm⁻¹): 3425, 3070, 2950, 1710, 1596, 1517, 1482, 1394, 1214, 1008, 1010, 840.
¹H-NMR (200MHz, CDCl₃): 7.40-7.05 (m,9H,2 arom.), 3.92 (s, 2H, CH₂), 2.448 (s,3H, CH₃, 2.297 (s, 3H, CH₃).

### Beispiel 5 (Referenzbeispiel)

### 2-[6-(5-Chlor2-thienyl)-3-methyl-7-phenyl-pyrrolo[2,1-b]thiazol -5-yl]-essigsäure

### nach Verfahren A3:

### A) 2-Benzyl-3-[2-(4-chlorthienyl)-2-oxo-ethyl]-4-methylthiazoliumbromid

aus 2-Benzyl-4-methyl-thiazol und 2-Brom-1-(5-chlor-2-thienyl)-ethanon analog Beispiel 2, Buchstabe A:

C₁₇H₁₅BrClNOS₂ (MG=428.8)
¹H-NMR (200MHz, DMSO-d6): 7,.91 (d, 1H, J=4.2Hz, thien.), 7.56 (s, 1H, thiaz.), 7.00 (s, 5H, arom.),6.88 (d, 1H, J=4.2Hz, thien.), 6.101 (s, 2H, CH₂CO), 4,369 (s, 2H, -CH₂Ph), 2.155 (s, 3H, CH₃).

### B) 6-(5-Chlor-2-thienyl)-3-methyl-7-phenyl-pyrrolo[2,1-b]thiazol

C₁₇H₁₂ClNS₂ (HG=329.87)
IR (cm⁻¹): 1591, 1522, 1395, 1160, 791, 762, 719, 694, 624. ¹H-NMR (200MHz, CDCl₃): 7,43-7,16 (m, 6H, arom.+pyrr.), 6.782 (d, 1H, J=3.8Hz, thien.), 6.658 (d, 1H, J=3.8Hz, thien.), 6.302 (s, 1H, thiaz., J=1.25 Hz,), 2,374 (q, 3H, J=1.25 Hz, CH₃).

### C) 2-[6-(5-Chlor-2-thienyl)-3-methyl-7-phenyl-pyrrolo[2,1-b]thiazo 1-5-yl]-2-oxo-essigsäurechlorid, (Variante B):

Zur Lösung von 6-(5-Chlor-2-thienyl)-3-methyl-7-phenyl-pyrrolo[2,1-b]thiazol (49.3 g, 0,15 Mol) in THF (300 mL) tropft man unter Eiskühlung Oxalylchlorid (22,5 g, 0,2 Mol) zu, rührt die Mischung bei RT. weitere 15' und versetzt vorsichtig mit Wasser (20 mL).
Das gebildete Säurechlorid wird nicht isoliert, sondern als Rohprodukt direkt umgesetzt mit Hydrazinhydrat / KOH in Diethylenglykol:

### D) 2-[6-(5-Chlor-2-thienyl)-3-methyl-7-phenyl-pyrrolo[2,1-b]thiazo l-5-yl]-essigsäure

Zu der unter Buchstabe C) erhaltenen wässrigen THF-Phase gibt man unter Eiskühlung Hydrazinhydrat (75 mL) und destilliert anschließend im Vakuum THF ab. Der abgekühlte Rückstand wird mit Diethylenglycol (120 mL) versetzt und KOH (120 g) zugegeben. Man erhitzt langsam auf 140 °C und hält bei dieser Temperatur bis am Wasserabscheider nur noch geringe Wasserreste übergehen und die Schaumbildung nachläßt. Die heiße Reaktionsmischung wird auf Eis gegossen (1 L) und mit HCl angesäuert (pH=2). ausgeschiedene Carbonsäure wird rasch mit Diethylether aufgenommen (1 L). Die Etherphase wird über Na₂SO₄ getrocknet und unter vermindertem Druck eingeengt. Die vom erstarrten Rückstand in der Wärme erhaltene Lösung in Toluol wird zur Kristallisation der Carbonsäure kaltgestellt: 17 g (29 %) cremefarbene Kristalle vom Schmelzpunkt182-183 °C.
C₁₉H₁₄ClNO₂S₂ (MG=387.91)
IR(cm⁻¹): 3425, 1694, 1596,
¹H-NMR (200MHz, CDCl₃): 7.40-7.1 (m, 5H, arom.), 6.871 (d, 1H, J=4.0Hz, thien.), 6.731 (d, 1H, J=4.0Hz, thien.), 6.306 (s, 1H, thiaz., J=1.0 Hz,), 4.043 (s, 2H, CH₂), 2,554 (q, 3H, J=1.0 Hz, CH₃).

### Beispiel 5a

### nach Verfahren I:

### 2-[6-(5-Chlor-2-thienyl)-3-methyl-7-phenyl-pyrrolo[2,1-b]thiazo 1-5-yl]-N-methylsulfonyl-acetamid

2-[6-(5-Chlor-2-thienyl)-3-methyl-7-phenyl-pyrrolo[2,1-b]thiazo 1-5-yl]-essigsäure (1,16 g, 3 mMol) wird in THF (30 mL) gelöst. Nach Zugabe von 1,1'-Carbonyldiimidazol (CDI, 1,04 g, 6 mMol) wird 1h gerührt. und anschließend das Na-salz des Methansulfonsäureamids (1,0 g, 10 mMol), hergestellt durch Zugabe von NaH (0,4 g, 80 % ig in Weißöl) zur Lösung des Amids in THF (30 mL) und Rühren (1h) in der Wärme (50 °C), zugetropft. Nach 20 h wird mit Wasser versetzt (200 mL) und mit HCl (3N)angesäuert (pH1). Der Etherextrakt (200mL) der wässrigen Phase wird getrocknet (Na₂SO₄) und eingeengt, der Rückstand aus Diisopropylether kristallisiert: 0,9 g, 65 %, Schmp. 203-204 °C.
C₂₀H₁₇ClN₂O₃S₃ (MG=465.01)
IR(cm⁻¹): 3430, 3175, 3161, 1678, 1596, 1466, 1438, 1341, 1133. ¹H-NMR (200MHz, CDCl₃): 8.2-7.7 (b, NH), 7.40-7.1 (m, 5H, arom.), 6.90 (d, 1H, J=4.0Hz, thien.), 6.69 (d, 1H, J=4.0Hz, thien.), 6.38 (s, 1H, thiaz., J=1.0 Hz,), 4.07 (s, 2H, CH₂), 3,271 (s, 3H, SO₂CH₃), 2,554 (q, 3H, J=1.0 Hz, CH₃).

### Beispiel 6

### 2-[6-(4-Chlorphenyl)-3,3-dimethyl-7-phenyl-2,3-dihydro-pyrrolo[ 2,1-b]thiazol-5-yl]-essigsäure (Syn.: 5-Carboxymethylen-6-(4-chlorphenyl)-3,3-dimethyl-7-phenyl-2,3-d ihydro-pyrrolo[2,1-b]thiazol)

### Verfahren A2)

### A) 3-Hydroxy-2-methyl-2-phenylacetamido-propan

Aus 2-Amino-2-methyl-propanol (0,12 Mol) und Phenylessigsäurechlorid (0,1Mol) in wässriger10 % iger NaOH (Schotten Baumann):
C₁₂H₁₇NO₂ (MG=207.3)
¹H-NMR (200MHz, CDCl₃): 7,40-7,20 (m, 5H, arom.), 5,67(b, 1H, NH), 3,53 (s, 4H, 2 CH₂), 2,10 (b, 1H, -OH), 1,205 (s, 6H, 2 CH₃).
¹³C-NMR (50, MHz, CDCl₃):24.5, 44.2, 56.2, 70.46, 127.4, 129.0, 134.8, 172.2.

### B) 2-Benzyl-4,4-dimethyl-4,5-dihydro-thiazol

Aus 3-Hydroxy-2-methyl-2-phenylacetamido-propan mit Phosphorpentasulfid analog zu einem bekannten Verfahren (Thewalt, K. , Renkhoff, G. , Fette, Seifen, Anstrichm., 1968, 70(9), 648-653.
C₁₂H₁₅NS (MG=205.3)
¹H-NMR (200MHz, CDCl₃)): 7,40-7,10 (m, 5H, arom.), 3,776 (s, 2H, CH₂), 3,053 (s, 2H, -CH₂), 1,372 (s, 6H, 2 CH₃).
¹³C-NMR (50, MHz, CDCl₃): 27.5, 40.9, 45.6, 78.2, 127.1, 128.6, 128.9, 136.2, 166.3.

### C) 2-(2-Benzyl-4,4-dimethyl-4,5-dihydro-thiazol-3-yl)-1-(4-chlor phenyl)-ethanonium-bromid 2-Benzyl-3-[2-[4-chlorphenyl)-2-oxo-ethyl]-3,3-dimethyl-2,3-dih ydro-thiazoliumbromid

nicht isoliert: analog zu Beispiel 1, Buchstabe B) wurde die gebildete zähe dunkle Masse des Rohproduktes durch Zugabe von Triethylamin direkt cyclisiert: SC (EE, Hexan1:5), Al₂O₃: 37% Ausbeute

### D) 6-(4-Chlorphenyl)-3,3-dimethyl-7-phenyl-2,3-dihydropyrrolo[2,1 -b]thiazol

C₂₀H₁₈ClNS (MG=339.89)
Schmelzpkt. 151-153 °C
IR (cm⁻¹):1597, 1515, 1478, 1364, 1180, 1084, 1009, 825, 750, 689.
¹H-NMR (200MHz, CDCl₃): 7.26-7.13 (m, 9H, 2 arom.), 6.753 (d, J=0.9Hz, 1H, pyrr.), 3.489 (d, J=1.9Hz, 2H, CH₂), 1.590 (s, 6H, 2 CH₃).
¹³C-NMR (50MHz, CDCl₃): 135.02, 134.59 131.40, 129.39 (2C), 128.31 (2C), 128.28 (2C), 128.25 (2C), 127.69, 126.73, 125.34, 114.27, 112.33, 62.54, 48.04, 26.27 (2C).

### E) 2-[6-(4-Chlorphenyl)-3,3-dimethyl-7-phenyl-2,3-dihydropyrrolo[ 2,1-b]thiazol-5-yl]-2-oxo-essigsäureethylester C₂₄H₂₂ClNO₃S (MG=439.96)

¹H-NMR (200MHz, CDCl₃): 2 isomere, cis/trans: 7.32-7.11 (m, 7H)7.00-6.89 (m, 2H), 3.683/3.676 (2 s, 2H, CH₂), 3,556/3,455 (q, 2H, J=7.1 Hz, CH₂), 1.865 (s, 6H, 2 CH₃), 1.090/1.046 (t, 3H, J=7.1Hz, CH₃).

### F) 2-[6-(4-Chlorphenyl)-3,3-dimethyl-7-phenyl-2,3-dihydropyrrolo[ 2,1-b]thiazol-5-yl]-essigsäure 5-Carboxymethylen-6-(4-chlorphenyl)-3,3-dimethyl-7-phenyl-2,3-d ihydro-pyrrolo[2,1-b]thiazol

C₂₂H₂₀ClNO₂S (MG=397.92)
IR (cm⁻¹):3423, 2932, 1714, 1617.5, 1346.3 1091 836,
¹H-NMR (200MHz, CDCl₃): 7.3-7.10 (m, 9H, 2 arom.), 3.82 (s, 2H, CH₂), 3.465 (d, J=1.7Hz, 2H, CH₂), 1.590 (s, 6H, 2 CH₃).

### Verfahren B2)

### A) 4-(4-Chlorphenyl)-1-(2-methyl-2-hydroxypropyl)-3-phenyl-1,5-dih ydro-pyrrol-2-on

nach Vorschrift Beispiel 7, Buchstabe A (s.u.):
C₂₀H₂₀ClNO₂ (MG=341.84)
IR(cm⁻¹): 3420, 1660, 1631, 1485, 1438, 1387, 1361, 1219, 1085, 1057, 1009, 827.
¹H-NMR (200MHz, CDCl₃): 7.354 (s, 5H, arom.), 7.30-7.18 (AA'BB', 4H, arom.), 5.30 (b, 1H, OH), 4.359 (s, 2H, CH₂), 3.845 (b, 2H, CH₂OH) 1.448 (s, 6H, 2 CH₃).

### B) Ringschluß analog Beispiel 7, Buchstabe C: Produkt identisch mit Produkt aus Buchstabe D) 1,2 g ,19 % Ausbeute.

### Beispiel 7

### Verfahren B2)

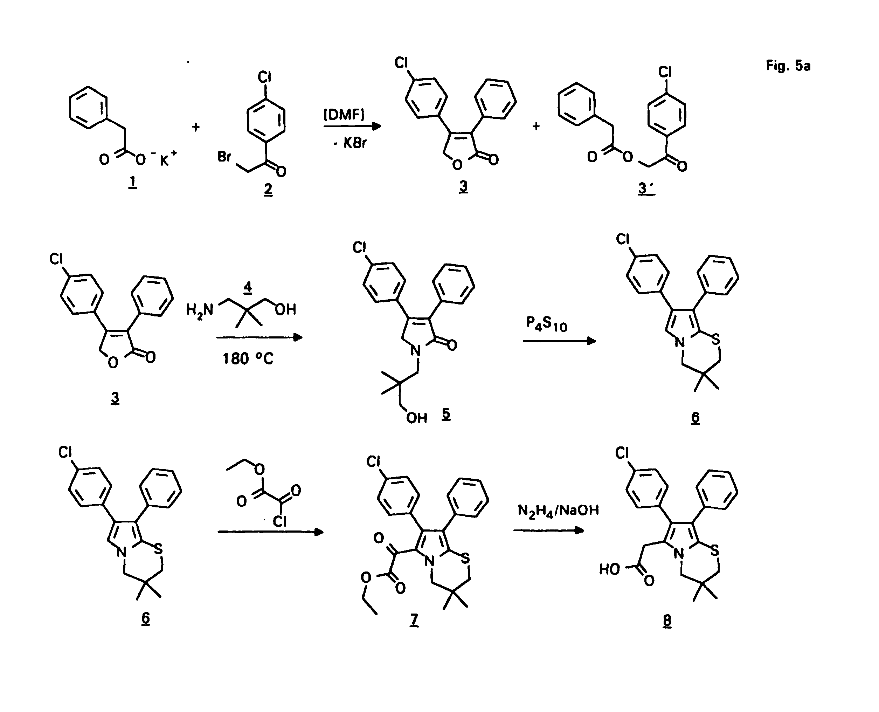

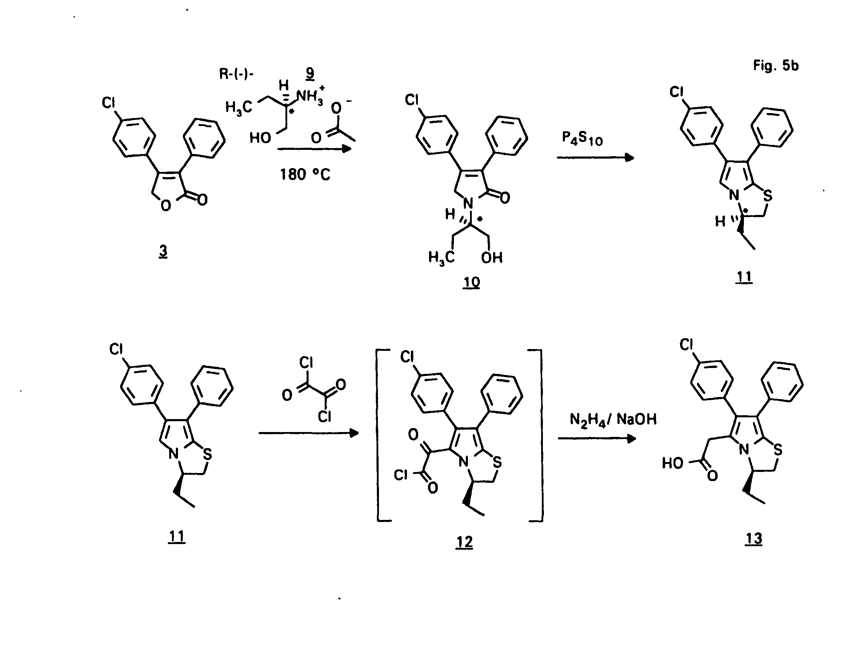

### 2-[6-(4-Chlorphenyl)-3,3-dimethyl-7-phenyl-3,4-dihydro-2H-pyrro lo[2,1-b]1,3-thiazin-5-yl]-essigsäure, 8 (vgl. Fig. 5)

### A) 4-(4-Chlorphenyl)-3-phenyl-2(5H)-furanon (3)

Hergestellt in Anlehnung an bekannte Verfahren (a: Rio, G. und Sekiz, B. Bull. Soc. Chim. Fr. 1976, 1491, 1495. b: Padwa, A. , Brookhart, T., Dehm, D. und Wubbels, G., J. Am. Chem. Soc. 1978, 100, 8247, 8259).

2-Brom-1-(4-chlorphenyl)-ethanon (2, 102,6, 0,44Mol) und Kaliumphenylacetat (1, 104,5g, 0.6 Mol werden in Dimethylformamid (600 mL) unter Rühren 4-Std. auf 80 °C erwärmt und anschließend abgekühlt. Nach Zugabe von Wasser (1L) wird mit Chloroform extrahiert (3mal 300 mL) und die Chloroformauszüge mit Wasser gewaschen anschließend getrocknet (Na₂SO₄) und zur Trockne eingedampft . Zur Kristallisation wird mit Diisopropylether (500mL) versetzt und die in der Kälte gebildeten Kristalle vom Schmelzpunkt. 111-113°C gesammelt (Ausbeute 92 g, 78 % bez. auf 2).

C₁₆H₁₁ClO₂ (MG=270.27)
IR (cm ⁻¹): 1740.0, 1156,0, 1028,0, 694.
¹H-NMR (200MHz, CDCl₃): 7.39 (s, 5H, arom.) 7.36 -7.20 (AA'BB', 4H, arom.), 5.154 (s, 2H, CH₂).

### B) 4-(4-Chlorphenyl)-1-(2,2-dimethyl-3-hydroxypropyl)-3-phenyl-1,5 -dihydro-pyrrol-2-on (5)

### Variante A:

Unter einer Schutzgasatmosphäre (N₂) wird ein Gemisch aus 4-(4-Chlorphenyl)-3-phenyl-2(5H)-furanon (3 26g, 0.08 Mol) und 3-Hydroxy-2,2-dimethyl-propylamin (4, Neopentanolamin, 80g, 0,8 Mol) unter Rühren mit aufgesetzter Destillationsbrücke auf 190-210 °C erhitzt. Nach 1 Std wird unter vermindertem Druck der Aminoalkoholüberschuß mit dem bei der Reaktion gebildeten Wasser abdestilliert. Nach Abkühlen wird der verbleibende Rückstand in Methylenchlorid aufgenommen und mit verd. Salzsäure (200 mL, 1N) extrahiert , mit Wasser (200 mL) gewaschen, getrocknet (Na₂SO₄) und bei vermindertem Druck eingeengt. Der verbleibende Rückstand wird mit Diisopropylether zur Kristallisation gebracht und die gebildeten Kristalle vom Schmelzpunkt 167 °C gesammelt (20.2g, 71%).

C₂₁H₂₂ClNO₂ (MG=355,87)
IR (cm ⁻¹): 3375, 1657, 1487, 1450, 1401, 1156, 1089,1047, 749. ¹H-NMR (200MHz, CDCl₃): 7,37 - 7.35 (m, 5H, arom.) 7.30 -7.15 (AA'BB', 4H, arom.), 4,73 (t, 1H, -OH), 4,410 (s, 2H, CH₂N), 3,39 (s, 2H, CH₂N), 3,262 (d, 2H, J= 7,5, CH₂OH), 0.994 (s, 6H, 2 CH₃).

### C) 7-(4-Chlorphenyl)-3,3-dimethyl-8-phenyl-3,4-dihydro-2H-pyrrolo[2,1-b]1,3-thiazin (6)

4-(4-Chlorphenyl)-1-(2,2-dimethyl-3-hydroxypropyl)-3-phenyl-1,5 -dihydro-pyrrol-2-on (5, 10,7g, 0.03 Mol) werden bei 170-180 °C Badtemp unter N₂-Schutzatmosphäre mit Phosphorpentasulfid (3.33g, 2,5 Äqiv., 0,015 Mol) geschmolzen, das in kleinen Portionen zugegeben wird bis die Gasentwicklung zum Stillstand kommt (1 Std.). Anschließend wird das abgekühlte Reaktionsgemisch mit Natronlauge (100 mL, 10%) alkalisiert und mit Chloroform extrahiert, die organische Phase mit Wasser gewaschen und getrocknet. Der nach Abdampfen von Chloroform verbleibende Rückstand kristallisiert aus Diisopropylether mit Schmelzpunkt 147-148°C (5.5g, 52%).

C₂₁H₂₀ClNS (MG=353,9)
IR (cm ⁻¹): 1596, 1510, 1481, 1381, 1168, 1089,1009, 833, 695. ¹H-NMR (200MHz, CDCl₃): 7,4 - 7.00 (m, 9H, arom.), 6,758 (s, 1H, pyrr.), 3,69 (s, 2H, CH₂N), 2,758 (s, 2H, CH₂S), 1,217 (s, 6H, 2 CH₃).
¹³C-NMR (50 MHz, CDCl₃): 25.9, 31.2, 37.8, 56.9, 120.6122.5, 125.7, 125.9, 128.0128.1, 1218.2, 128.25, 129.2, 129.7, 130.2, 131.2, 13.5, 134.0 134.7, 135.2.

### D) 2-[7-(4-Chlorphenyl)-3,3-dimethyl-8-phenyl-3,4-dihydro-2H-pyrro lo[2,1-b]1,3-thiazin-6-yl]-2-oxo-essigsäureethylester (7) analog Umsetzung in Beispiel 2, Buchstabe C) mit Oxalsäureethylesterchlorid

C₂₅H₂₄ClNO₃S (MG= 453.99) Gemisch zweier Isomere: cis/trans im Verhältnis 4:6
Isomer a: Schmp.: 169 °C:
IR (cm⁻¹): 1733, 1611, 1358, 1251, 1193, 699.
¹H-NMR (200MHz, CDCl₃): 7.30-6.95 (m, 9H, arom.), 4.335 (s, 2H, CH₂), 3.552 / 3.470 (q, 2H, CH₂, J=7.2Hz), 2.821/2.812 (s,2H, CH₂), 1.259 (s, 6H, 2 CH₃), 1,080 / 1.036 (t, 3H, CH₃, J=7.2 Hz).

### E) 2-[7-(4-Chlorphenyl)-3,3-dimethyl-8-phenyl-3,4-dihydro-2H-pyr rolo[2,1-b]1,3-thiazin-6-yl]-essigsäure (8) analog Beispiel 2, Buchstabe D:

C₂₃H₂₂ClNO₂S (MG=411.954)
Schmp.: 148 °C
IR (cm ⁻¹): 1702, 1596, 1220, 1512, 832, 698.
¹H-NMR (200MHz, CDCl₃): 7.35-7.00 (9H, m, 2 Ar.), 5,7 (b, COOH), 3.639 (s,2H, CH₂), 2.747 (s, 2H, CH₂), 1.217 (s, 6H, 2 CH₃).

### Beispiel 8

### Verfahren B2)

### (R)-2-[6-(4-Chlorphenyl)-3-ethyl-7-phenyl-2,3-dihydro-pyrrolo[2 ,1-b]thiazol-5-yl]-essigsäure (13, vgl. Fig. 5)

### A) (R)-4-(4-Chlorphenyl)-1-(2-hydroxybutyl)-3-phenyl-1,5-dihydro -pyrrol-2-on (10)

### Variante 2:

Analog zur Umsetzung von 3,4-Diphenyl-2(5H)-furanone mit Ammoniumacetat zu 3,4-Diphenyl-1,5-dihydro-pyrrol-2-on bei Rio, G. und Sekiz, B.(s.o.):
Zur Schmelze von (R)-1-Hydroxy-2-butyl-ammoniumacetat (9), hergestellt durch vorsichtiges Mischen von R-(-)-2-Amino-1-butanol (35,6g, 0,4 Mol) mit äquimolaren Mengen an Eisessig (24g, 0,4 Mol), wird 4-(4-Chlorphenyl)-3-phenyl-2(5H)-furanon (3, 11 g, 0.04 Mol) gegeben und das Gemisch an einer Destillationsbrücke unter einer Schutzgasatmosphäre auf 180 - 200 °C erhitzt, gebildete freie Essigsäure wird abdestilliert. Nach 1 h .läßt man abkühlen und nimmt die erstarrte Masse in Methylenchlorid (150mL) auf. Mit Wasser (200 mL), verd. NaHCO₃ (100mL, 10%), HCl (50 mL, 1N) und Wasser (100mL) wird ausgewaschen, getrocknet (Na₂SO₄) und bei vermindertem Druck die Lösung eingeengt. der verbleibende Rückstand wird aus Diethylether kristallisiert oder durch Säulenchromatographie (SiO₂, Diethylether) gereinigt: (6 g, 44%).

C₂₀H₂₀ClNO₂ (MG=341.84)
Schmp. 139 -140 °C
IR(cm⁻¹):3378, 2964, 1660, 1497, 1458, 1402, 1364, 1231, 1091, 824, 777, 751, 742.
¹H-NMR (200MHz, CDCl₃): 7.40-7.15 (m, 9H, arom.), 4.407 / 4,240 (AB, J_{AB}= 19.4Hz)4.4-4.0 / 4.0-3.7 (m, 3H, CH-CH₂), 3.1 (b, 1H, OH), 2.0-1.6 (m, 2H, CH₂), 0.991 (t, 3H, CH₃).

### B) (R)-2-[6-(4-Chlorphenyl)-3-ethyl-7-phenyl-2,3-dihydropyrrolo[2 ,1-b]thiazol (11)

(R)-4-(4-Chlorphenyl)-1-(2-hydroxybutyl)-3-phenyl-1,5-dihydro-p yrrol-2-on (10, 10,65g, 0.03 Mol) werden bei 170-180 °C Badtemp unter N₂-Schutzatmosphäre mit Phosphorpentasulfid (6.3g, portionsweise) geschmolzen bis die Gasentwicklung zum Stillstand kommt (1 Std.). Anschließend wird das abgekühlte Reaktionsgemisch mit Natronlauge (100 mL, 10%) alkalisiert und mit Chloroform extrahiert, die organische Phase mit Wasser gewaschen und getrocknet. Der nach Abdampfen von Chloroform verbleibende Rückstand kristallisiert langsam aus: (3.9g, 37%) Schmp. 58 - 60 °C.
C₂₀H₁₈ClNS (MG=339.89)
IR(cm⁻¹): 2926, 1700, 1594, 1520, 1386, 1170, 1084.850, 700. ¹H-NMR (200MHz, CDCl₃): 7.50-6.90 (m, 9H, arom.), 6.807 (s, 1H, pyrrol.), 4.270 (m, 1H, CH), 3.9-3.7 / 3.5-3.3 (AB-Teil im ABXsystem CH-CH₂-, 2H), .2.3-1.6 (m, 2H, CH₂), 1.091 (t, 3H, CH₃).

### C) (R)-2-[6-(4-Chlorphenyl)-3-ethyl-7-phenyl-2,3-dihydropyrrolo[2 ,1-b]thiazol-5-yl]-2-oxo-essigsäureethylester gelbe Kristalle aus Diisopropylether, 9 g, 37 %: Schmp.:

C₂₄H₂₂ClNO₃S (MG=439.9)
¹H-NMR (200MHz, CDCl₃):7.35-7.10 (m, 7H, arom.)7.05-6.95 (m, 2H, arom.) 5.34 (m, 1H, CH), 4.10-3.95 / 3.65-3.45 (m, 2H, AB-teil des CHCH₂-Systems), 3.60 (q, 2H, J=7.2 Hz, CH₂), 2.1-1.7 (m, 2H, CH₂), 1.074 (t, 3H, CH₃), 1.047 (t, 3H, CH₃).

### D) (R)-2-[6-(4-Chlorphenyl)-3-ethyl-7-phenyl-2,3-dihydropyrrolo[2 ,1-b]thiazol-5-yl]-essigsäure

C₂₂H₂₀ClNO₂S (MG=397.93)
IR (cm⁻¹): 3427, 1707, 1601, 1522, 1362, 1487, 1459, 1091, 835,700.
¹H-NMR (200MHz, CDCl₃): 7.5-6.9 (m, 9H, arom.), 4.52 (m, 1H, CH), 4.05-3.85 / 3.5-3.35 (m, AB-Teil des ABX-Systems CHCH₂-), 3.65-3.55 (m, CH₂-CO), 2.2-1.6 (m, 2H, CHCH₂CH₃), 1.052 (t, 3H, J= 7,4 Hz, CH₃).

### E) (S)-2-[6-(4-Chlorphenyl)-3-ethyl-7-phenyl-2,3-dihydropyrrolo[2 ,1-b]thiazol-5-yl]-essigsäure

auf analoge Weise wurde aus (S)-(+) 1-Hydroxy-2-butylamin das Enantiomer zu Substanz D) erhalten:
¹H-NMR identisch mit (R)-Enantiomer-Spektrum:
IR (cm ⁻¹): 3427, 2965, 2931, 1707, 1601, 1522, 1091. Schmp.: 102-104 °C

### Beispiel 9

### (R,S)-6-(4-Chlorphenyl)-2-methyl-7-phenyl-2,3-dihydro-pyrrolo[2 ,1-b]thiazol

### A) 4-(4-Chlorphenyl)-1-[(S)-1-hydroxy-2-propyl]-3-phenyl-1,5-dih ydro-pyrrol-2-on hergestellt aus 3 nach Verfahren B2 analog Beispiel 8 (vgl. Fig 5), 10,6 g, 64%:

C₁₉H₁₈ClNO₂ (MG=327.81):
Schmp.: 97.5-98.5 °C (aus Diisopropylether)
IR (cm⁻¹): 3395, 1672, 1485, 1451, 1399, 1090, 826, 695.
¹H-NMR (200MHz, CDCl₃): 7.40-7.30 (m, 5H, arom.), 7.30-7.18 (AA'BB', 4H, arom.), 4.437 (s, 2H, CH₂), 4.16 (m 1H, CH), 3.70-3.50 (m, 2H, AB-Teil des ABX-Systems CHCH₂), 3.19 (d, 1H, OH, J= 4.3 Hz), 1.273 (d, 3H, CH₃, J= 6.3 Hz).

### B) (R,S)-6-(4-Chlorphenyl)-2-methyl-7-phenyl-2,3-dihydropyrrolo [2,1-b]thiazol nach SC-Reinigung auf SiO₂ mit EE/Hexan (2:8): zähe Masse, keine Kristallisation, 4,8g, 50% Racemat).

C₁₉H₁₆ClNS (MG=325.86)
IR (cm⁻¹): 1697, 1594, 1516, 1479, 1440, 1391, 1369, 1087, 1008, 829, 694.
¹H-NMR (200MHz, CDCl₃): 7.4-7.0 (m, 9H, 2 arom.), 6.79 (s, 1H, pyrr.), 4.5-4,2 / 4.1-3.7 (m, 2H, AB-Teil des ABX-Systems CHCH₂), 3,82 (m 1H, CH), 1.61-1.54 (m, 3H, CH₃, J= 6.3 Hz, J= 0.6 Hz).

### Beispiel 10

### 2-[6-(4-Chlorphenyl)-3,3-dimethyl-1,1-dioxo-7-phenyl-2,3-dihydr o-pyrrolo[2,1-b]thiazol

6-(4-Chlorphenyl)-3,3-dimethyl-7-phenyl-2,3-dihydro-pyrrolo[2,1 -b]thiazol, hergestellt nach Verfahren B2 wie in Beispiel 6 beschrieben (340mg, 1mMol) und gelöst in Methylenchlorid, (50 mL) wird in die getrocknete (Na₂SO₄)-Lösung von m-Chlorperbenzoesäure (850 mg, 55%, 2,5 mMol) in Methylenchlorid getropft. Unter Rückflußkochen wird gerührt bis nach dünnschichtchromato-graphischer Kontrolle (SiO₂, CH₂Cl₂) der Umsetzung kein Edukt (Rf =0,95) mehr nachweisbar ist und primär gebildetes Sulfoxid (Rf =0,1) weitestgehend in Sulfon (RF=0,3) umgewandelt wurde (16 h). Die Reinigung erfolgt über Säulenchromatographie auf neutralem Al₂O₃ (Akt.II) mit Diethylether als Eluens: Sulfon (Rf=0,75, 230 mg, Sulfoxid (Rf= 0,5, 30 mg).

C₂₀H₁₈ClNO₂S (MG= 371,89)
¹H-NMR (200MHz, CDCl₃): 7,36-7,22 (m,5H, arom.), 7,28-7,20 /7,18-7,10 (AA'BB', 4H, arom.), 6,91 (s, 1H, pyrr.), 3,755 (s, 2H, CH₂SO₂), 1,804 (s, 6H,2 CH₃).

### Beispiel 11

### (R,S)-2-[6-(4-Chlorphenyl)-3,3-dimethyl-1-oxo-7-phenyl-2,3-dihy dro-pyrrolo[2,1-b]thiazol

aus 2-[6-(4-Chlorphenyl)-3,3-dimethyl-7-phenyl-2,3-dihydropyrrolo[ 2,1-b]thiazol, erhalten nach Verfahren B2)

Analog zu Beispiel 10: Das Sulfoxid wird überwiegend gebildet, wenn geringere Mengen m-Chlorperbenzoesäure eingesetzt werden (1,2 Äquivalente, 400 mg) und bei tieferen Temperaturen gearbeitet wird (-20 °C, 2h).

Die Reinigung erfolgt wie unter Beispiel 10 beschrieben mittels SC auf neutralem Al₂O₃ (Akt.II) mit Diethylether als Eluens:: Edukt (20 mg), Sulfon (80 mg), Sulfoxid (170 mg).

C₂₀H₁₈ClNOS (MG=355,89)
¹H-NMR (200MHz, CDCl₃): 7,45-7,30 (m,5H, arom.), 7,35-7,20 /7,20-7,15 (AA'BB', 4H, arom.), 6,95 (s, 1H, pyrr.), 3,60 (/3,48 (AB, 2H, J_{AB}=13,6 Hz, CH₂SO), 1,937 (s, 3H,CH₃), 1,713 (s, 3H, CH₃).

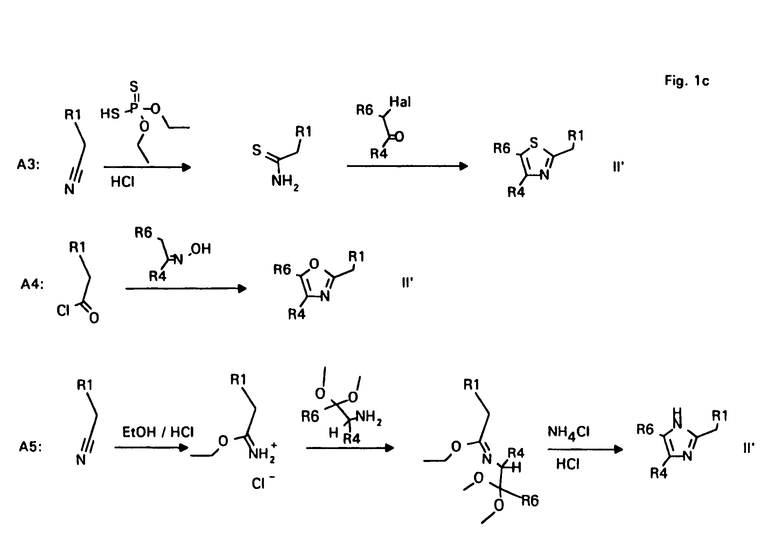

In analoger Weise wurden die zusammengestellten Verbindungen nach den entsprechenden Verfahren der Fig. 1 bis 5 erhalten. Die Verbindungen der Beispiele 1 bis 11 wurden ebenfalls in die Tabelle aufgenommen.

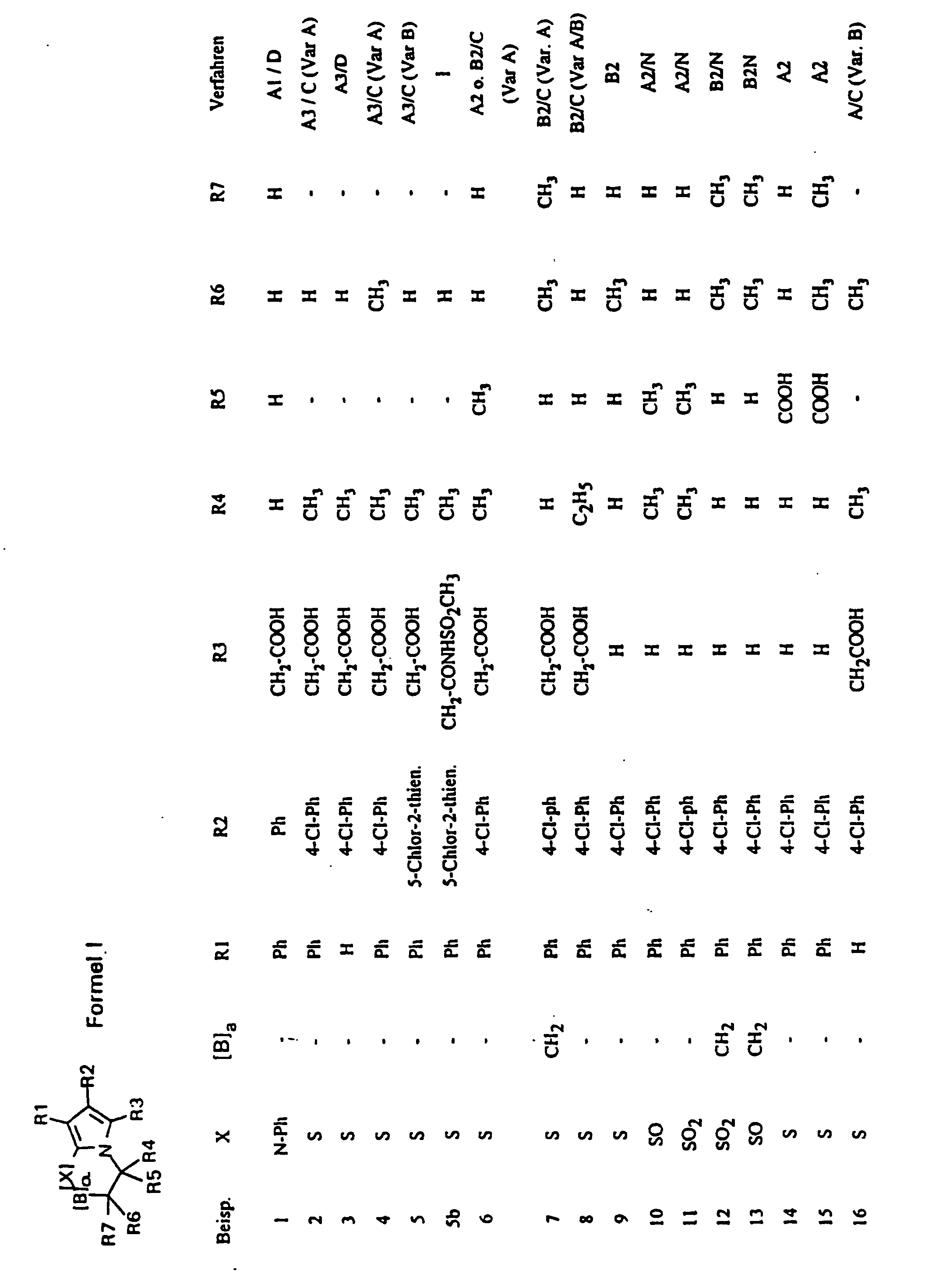

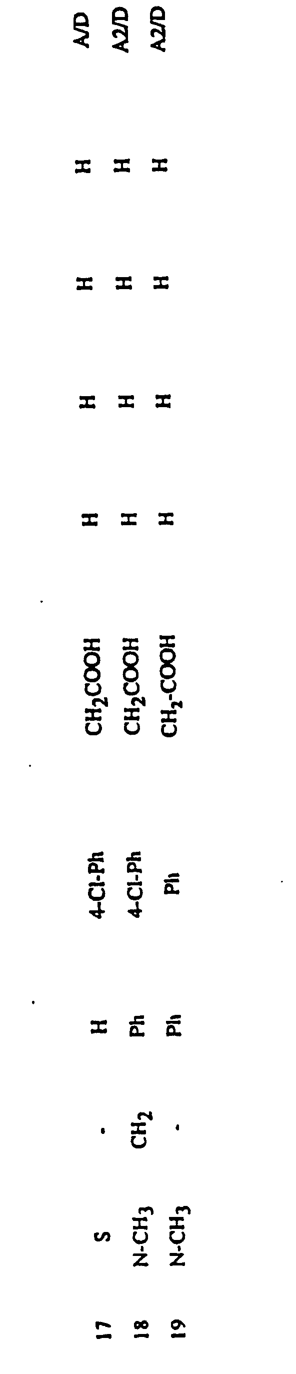

## Patentansprüche

1. Heterocyclische Verbindungen der Formel I: 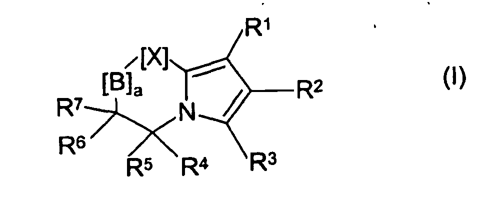 worin
zwei der Reste R¹, R² und R³, die gleich oder verschieden sein können, für einen Phenyl- oder Naphthylrest, der gegebenenfalls einen oder zwei Substituenten aufweist, die unter Halogen, Pseudohalogen, CF₃, NO₂, OH, C₁-C₈-Alkoxy, OCF₃, C₁-C₈-Alkyl oder Phenoxy ausgewählt sind, oder einen aromatischen heterocydtschen Rest, der ausgewählt ist unter einem Pyrrol-, Imidazol-, Thiazol-, Thiadiazol-, Oxazol-, Isoxazol-, Pyridin- oder Chinolinrest und der gegebenenfalls durch Halogen, CF₃, C₁-C₈-Alkyl oder C₁-C₈-Alkoxy substituiert ist, stehen und
der dritte der Reste R¹, R² und R³ für H, CHO, CO₂H, COCO₂H, COCO₂ C₁-C₈-Alkyl oder A - Y steht,
A für C₁-C₈-Alkylen oder C₂-C₈-Alkenylen steht,
Y für CO₂H, SO₃H, OPO(OH)₂, PO(OH)₂ oder Tetrazolyl, CHO oder OH steht,
R⁴, R⁵, R⁶ und R⁷, die gleich oder verschieden sein können, für H oder C₁-C₈ -Alkyl stehen
X für O, S, SO, SO₂ oder NR¹⁰ steht, wobei R¹⁰ für H, C₁-C₈-Alkyl oder Phenyl steht,
B für CR¹¹R¹² steht, wobei R¹¹ und R¹², die gleich oder verschieden sein können, für H oder C₁-C₈-Alkyl stehen und
a für 0, 1 oder 2 steht, und
deren optische Isomere, Salze und C₁-C₈-Alkyl-, Pivaloyloxymethyl-, Acetoxymethyl-, Phthalidyl-, Indanyl- und Methoxymethylester, ausgenommen eine Verbindung der Formel, worin R¹ für H, R² und R³ für Phenyl, a für O, X für NC₂H₅ und R⁴, R⁵, R⁶ und R⁷ für H stehen.

2. Verbindungen nach Anspruch 1 der Formel I, worin zwei der Reste R¹, R² und R³ unabhängig voneinander für einen Phenylrest, einen durch ein bis drei Halogenatome substituierten Phenylrest oder einen wie in Anspruch 1 definierten aromatischen heterocyclischen Rest, der gegebenenfalls durch ein Halogenatom substituiert sein kann, stehen und der dritte der Reste R¹, R² und R³ für A - Y steht,
A für C₁-C₈-Alkylen steht und
Y für CO₂H, SO₃H, CHO oder COCO₂H steht.

3. Verbindungen nach Anspruch 1 der Formel 1, worin zwei der Reste R¹, R² und R³ unabhängig voneinander für einen Phenylrest, einen durch ein oder zwei Halogenatome substituierten Phenylrest oder einen wie in Anspruch 1 definierten aromatischen heterocyclischen Rest, der gegebenenfalls durch ein Halogenatom substituiert sein kann, stehen,
der dritte der Reste R¹, R² und R³ für ein Wasserstoffatom steht, R⁴, R⁵, R⁶ und R⁷ die in Anspruch 1 angegebenen Bedeutungen besitzen;
X für S, SO oder SO₂ oder NR¹⁰ steht, wobei R¹⁰ für H, C₁-C₈-Alkyl oder Phenyl steht,
B für CH₂ steht und
a für 0, 1 oder 2 steht

4. Verbindungen nach Anspruch 2 der Formel I, worin R¹ für Phenyl steht und R² für Phenyl oder halogen-substituiertes Phenyl steht.

5. Verbindungen nach Anspruch 1 der Formel I, worin einer oder zwei der Reste R¹, R² und R³ für einen wie in Anspruch 1 definierten, gegebenenfalls substituierten aromatischen, heterocyclischen Rest stehen.

6. Verbindungen nach einem der vorhergehenden Ansprüche der Formel 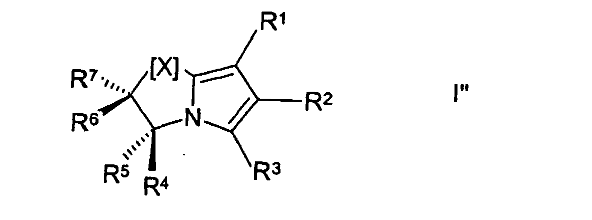 worin X, R¹, R², R³, R⁴, R⁵, R⁶ und R⁷ die in einem der vorhergehenden Ansprüche angegebenen Bedeutungen besitzen.

7. Verbindungen nach Anspruch 6 der Formel I" worin X für S, SO oder SO₂ steht, R¹ für Phenyl steht, R²
für 4-Chlorphenyl steht und R³ eine der oben genannten Bedeutungen, vorzugsweise A-Y, wie insbesondere -CH₂COOH, besitzt und die Reste R⁴, R⁵, R⁶ und R⁷ für H oder Methyl stehen.

8. Pharmazeutisches Mittel, enthaltend wenigstens eine Verbindung der Formel 1, worin einer der Reste R¹, R² und R³ für A - Y steht, worin Y für CO₂H oder SO₃H steht und die beiden anderen der Reste R¹, R² und R³, A, R⁴, R⁵, R⁶, R⁷, X, B und a die in Anspruch 1 angegebenen Bedeutungen besitzen, oder ein optisches Isomer, pharmazeutisch verträgliches Salz oder einen C₁-C₈-Alkyl-, Pivaloyloxymethyl-, Acetoxymethyl-, Phthalidyl-, Indanyl- und Methoxymethylester davon, gegebenenfalls in Kombination mit pharmazeutisch verträglichen Träger- und/oder Zusatzstoffen.

9. Verwendung wenigstens einer Verbindung der Formel I, wie in Anspruch 8 definiert, zur Herstellung eines pharmazeutischen Mittels zur Prävention von allergisch induzierten Erkrankungen oder zur Behandlung von Erkrankungen des rheumatischen Formenkreises.

10. Verfahren zur Herstellung der Verbindungen nach einem der Ansprüche 1 bis 7, **dadurch gekennzeichnet, daß** man
eine Verbindung der allgemeinen Formel II: 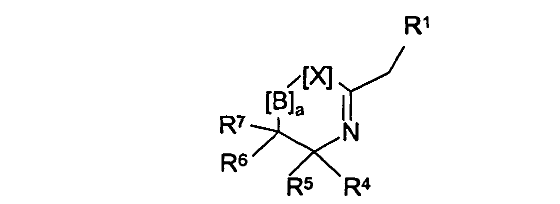
mit einer Verbindung der allgemeinen Formel III: 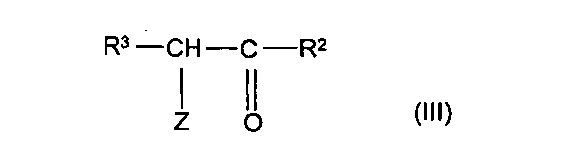 wobei in den obigen Formeln zwei der Reste R¹, R² und R³ die in Anspruch 1 angegebenen Bedeutungen besitzen und der dritte für ein Wasserstoffatom steht und Z für Cl oder Br steht, in einem Ether oder aromatishen kohlenwasserstoft zu einem intermediär gebildeten Quaternisierungsprodukt umseht und das Quaternisierungsprodukt in einem inerten Lösungsmittel mit Triethylamin behandelt, wobei man eine Verbindung der allgemeinen Formel la: 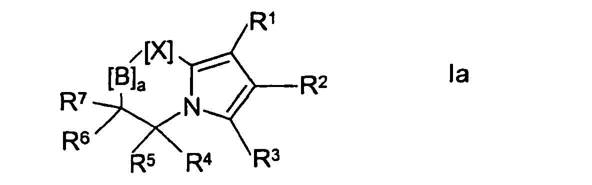 worin R¹ bis R⁷, B, a und X die oben angegebenen Bedeutungen besitzen, erhält.

11. Verfahren nach Anspruch 10, **dadurch gekennzeichnet, dass** man zur Herstellung der Verbindungen der Formel I, worin der dritte der Reste R₁, R₂ und R₃ für CH₂COOH, CH₂COOC₁-C₈-Alkyl
oder COCO₂H steht, eine Verbindung der in Anspruch 10 definierten Formel Ia
a) mit Oxalylchlorid zu einer Verbindung der Formel I umsetzt, in der einer der Reste R¹, R² und R³ für COCO₂H steht, und gewünschtenfalls diese Verbindung mit einem Reagens behandelt, das zur Reduktion der Ketogruppe der Ketocarbonsäure zu einer CH₂-Gruppe geeignet ist, so daß man eine Verbindung der Formel I erhält, in der einer der Reste R¹, R² und R³ für CH₂CO₂H steht,
b) mit einem Diazoessigsäurealkylester zu einer Verbindung der Formel I umsetzt, in der einer der Reste R¹, R² und R³ für CH₂COOC₁-C₈-Alkyl steht, und gewünschtenfalls die erhaltene Verbindung einer Esterspaltung unterwirft, um eine Verbindung der Formel I zu erhalten, in der einer der Reste R¹, R² und R³ für CH₂CO₂H steht, oder
c) mit Chloral zu einer Verbindung der Formel I umsetzt, in der einer der Reste R¹, R² und R³ für -CH(OH)CCl₃ steht, und die erhaltene Verbindung in ein aktiviertes Derivat überführt und dieses mit Dithionit zu einer Verbindung der Formel I reduziert, in der einer der Reste R¹, R² und R³ für CH₂COOH steht.

12. Verfahren zur Herstellung der Verbindungen gemäß Anspruch 1, worin X für O oder S steht, **dadurch gekennzeichnet, daß** man eine Verbindung der Formel VI: 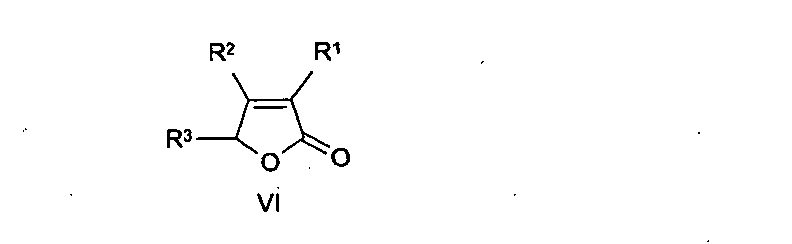 worin R¹, R² und R³ die in Anspruch 1 angegebenen Bedeutungen besitzen, mit einer Verbindung der Formel 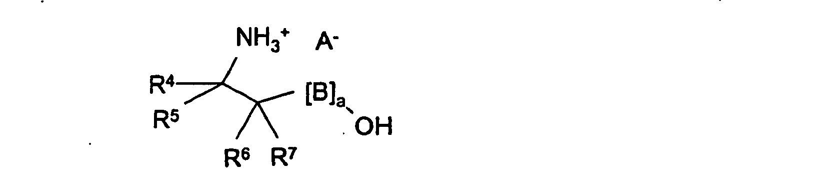 worin A' für ein Anion steht, B, a und R⁴ bis R⁷ die in Anspruch 1 genannten Bedeutungen besitzen, zu einer Verbindung der Formel VII: 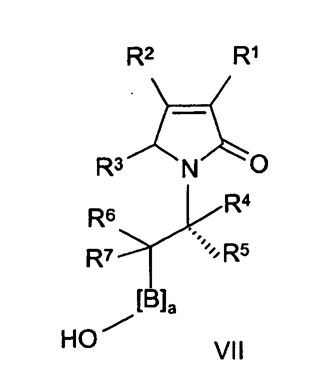 umsetzt und die Verbindung der Formel VII zu einer Verbindung der Formel 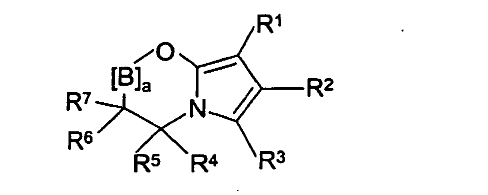 cyclisiert, vorzugsweise mit Hilfe von Polyphosphorsäure, oder
eine Verbindung der Formel VII in Anwesenheit von Phosphorpentasulfid zu einer Verbindung der Formel 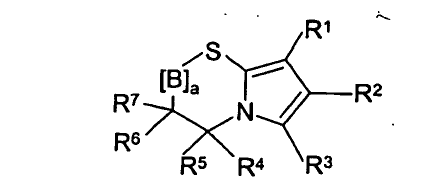 cyclisiert.

## Claims

1. Heterocyclic compounds of formula I: 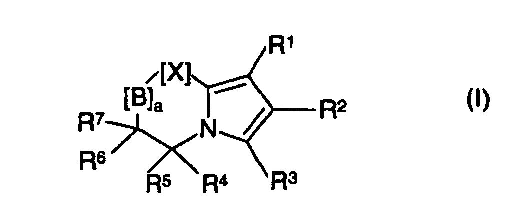 wherein two of the radicals R¹, R² and R³, which may be the same or different, represent a phenyl or naphthyl radical which optionally has one or two substituents selected from halogen, pseudohalogen, CF₃, NO₂, OH, C₁-C₈- alkoxy, OCF₃, C₁-C₈-alkyl or phenoxy, or an aromatic heterocyclic radical selected from a pyrrole, imidazole, thiazole, thiadiazole, oxazole, isoxazole, pyridine or quinoline radical and which optionally is substituted by halogen, CF₃, C₁-C₈-alkyl or C₁-C₈-alkoxy and
the third of the radicals R¹, R² and R³ is H, CHO, CO₂H, COCO₂H, COCO₂ C₁-C₈-alkyl or A - Y,
A is C₁-C₈-alkylene or C₂-C₈-alkenylene,
Y is CO₂H, SO₃H, OPO(OH)₂, PO(OH)₂ or tetrazolyl, CHO or OH,
R⁴, R⁵ ,R⁶ and R⁷ which may be the same or different are H or C₁-C₈-alkyl,
X is O, S, SO, SO₂ or NR¹⁰ where R¹⁰ is H, C₁-C₈-alkyl or phenyl,
B is CR¹¹R¹² where R¹¹ and R¹² which may be the same or different are H or C₁-C₈-alkyl and
a is 0, 1 or 2, and
their optical isomers, salts and C₁-C₈-alkyl, pivaloyloxymethyl, acetoxymethyl, phthalidyl, indanyl and methoxymethyl esters, with the exception of a compound of the formula wherein R¹ is H, R² and R³are phenyl, a is 0, X is NC₂H₅ and R⁴, R⁵, R⁶ and R⁷ are H.

2. The compounds of claim 1 having formula I wherein two of the radicals R¹, R² and R³ independently of each other are a phenyl radical, a phenyl radical which is substituted by one to three halogen atoms or an aromatic heterocyclic radical which is defined as in claim 1 and which is optionally substituted by a halogen atom, and
the third of the radicals R¹, R² and R³ is A-Y,
A is C₁-C₈-alkylene and
Y is CO₂H, SO₃H, CHO or COCO₂H.

3. The compounds of claim 1 having formula I wherein two of the radicals R¹, R² and R³ independently of each other are a phenyl radical, a phenyl radical substituted by one or two halogen atoms or an aromatic heterocyclic radical which is defined as in claim 1 and which optionally is substituted by a halogen atom,
the third of radicals R¹, R² and R³ is hydrogen,
R⁴, R⁵, R⁶ and R⁷ have the meanings given in claim 1;
X is S, SO or SO₂ or NR¹⁰ where R¹⁰ is H, C₁-C₈-alkyl or phenyl,
B is CH₂, and
a is 0, 1 or 2.

4. The compounds of claim 2 having formula I wherein R¹ is phenyl and R² is phenyl or halogen substituted phenyl.

5. The compounds of claim 1 having formula I wherein one or two of the radicals R¹, R² and R³ are an optionally substituted aromatic heterocyclic radical as defined in claim 1.

6. The compounds according to any one of the preceding claims having the formula 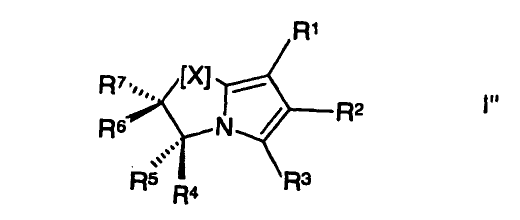 wherein X, R¹, R², R³, R⁴, R⁵, R⁶ and R⁷ have the meanings given in one or the preceding claims.

7. The compounds of claim 6 having formula I" wherein X is S, SO or SO₂, R¹ is phenyl, R² is 4-chlorophenyl and R³ has one of the above-mentioned meanings, preferably A-Y, in particular-CH₂COOH, and the radicals R⁴, R⁵, R⁶ and R⁷ are H or methyl.

8. A pharmaceutical composition containing at least one compound of formula I wherein one of the radicals R¹, R² and R³ is A-Y wherein Y is CO₂H or SO₃H and the two other radicals of R¹, R² and R³, A, R⁴, R⁵, R⁶, R⁷, X, B and a have the meanings given in claim 1 or an optical isomer, pharmaceutically acceptable salt or a C₁-C₈-alkyl, pivaloyloxymethyl, acetoxymethyl, phthalidyl, indanyl and methoxymethyl ester thereof, optionally in combination with pharmaceutically acceptable carriers and/or excipients.

9. The use of a compound of formula I as defined in claim 8 for the production of a pharmaceutical composition for the prevention of allergically induced diseases or for the treatment of diseases of the rheumatic type.

10. A process for producing the compounds according to one of claims 1 to 7, **characterized in that** a compound of the general formula II: 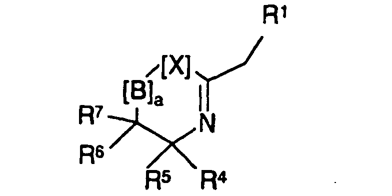 is reacted with a compound having the general formula III: 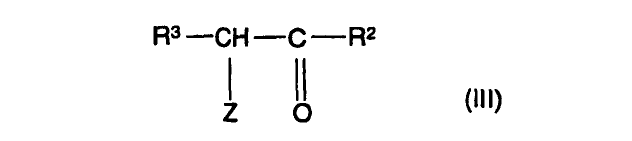 where in the above formulas, two of the radicals R¹, R² and R³ have the meanings indicated in claim 1 and the third is a hydrogen atom and Z is Cl or Br, in an ether or aromatic hydrocarbon to an intermediate quaternization product and the quatemization product is treated with triethylamine in an inert solvent to form a compound with the general formula la: 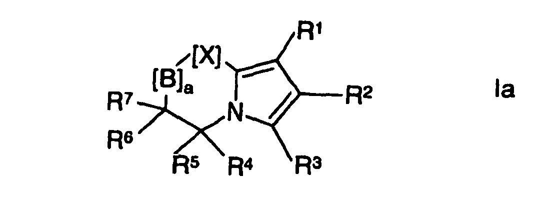 in which R¹ to R⁷, B, a, and X have the meanings given above.

11. The process according to claim 10, **characterized in that** for the production of the compounds of formula I, in which the third of the radicals R¹, R² and R³ is CH₂COOH, CH₂COOC₁-C₈-alkyl, or COCO₂H, a compound of formula la as defined in claim 10
a) is reacted with oxalyl chloride to form a compound of formula I, in which one of the radicals R¹, R² and R³ is COCO₂H, and if desired, this compound is treated with a reagent that is suitable for the reduction of the keto group of the ketocarboxylic acid to a CH₂ group so that a compound of formula I is obtained, in which one of the radicals R¹, R² and R³ is CH₂CO₂H,
b) is reacted with a diazoacetic acid alkyl ester to form a compound of formula I, in which one of the radicals R¹, R² and R³ is CH₂COOC₁-C₈-alkyl, and, if desired, the compound obtained is subjected to an ester cleavage in order to obtain a compound of formula I, in which one of the radicals R¹, R² and R³ is CH₂CO₂H, or
c) is reacted with chloral to form a compound of formula I, in which one of the radicals R¹, R² and R³ is -CH(OH)CCl₃ and the compound obtained is converted into an activated derivative and this is reduced with dithionite to a compound of formula I, in which one of the radicals R¹, R² and R³ is CH₂COOH.

12. A process for producing the compounds of claim 1, in which X is O or S, **characterized in that** a compound of formula VI: 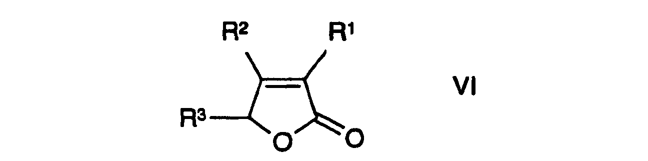 in which R¹, R² and R³ have the meanings indicated in claim 1, is reacted with a compound of the formula 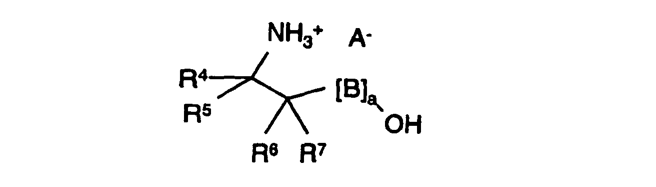 in which A⁻ is an anion, B, a, and R⁴ to R⁷ have the meanings mentioned in claim 1, to form a compound of formula VII: 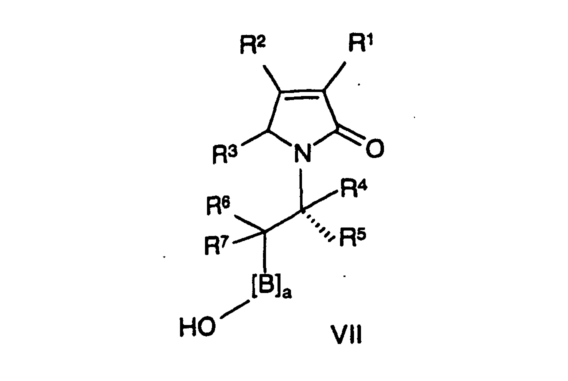 and the compound of formula VII is cyclized into a compound of the formula 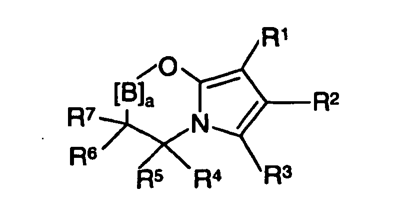 preferably with the aid of polyphosphoric acid, or a compound of formula VII, in the presence of phosphorus pentasulfide, is cyclized into a compound of the formula

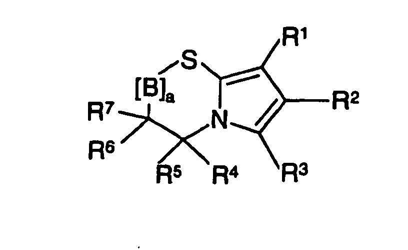

## Revendications

1. Composés hétérocycliques de formule I : 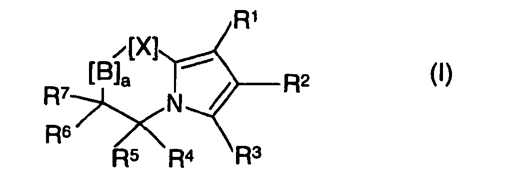 où
deux des groupements R¹, R² et R³, qui peuvent être identiques ou différents, représentent un groupement phényle ou naphtyle, qui comporte éventuellement un ou deux substituants qui sont choisis parmi halogène, pseudohalogène, CF₃, NO₂, OH, C₁-C₈-alcoxy, OCF₃, C₁-C₈-alkyle ou phénoxy, ou un groupement hétérocyclique aromatique qui est choisi parmi un groupement pyrrole, imidazole, thiazole, thiadiazole, oxazole, isoxazole, pyridine ou quinoléine et qui est éventuellement substitué par halogène, CF₃, C₁-C₈-alkyle ou C₁-C₈-alcoxy, et
le troisième des groupements R¹, R² et R³ représente H, CHO, CO₂H, COCO₂H, COCO₂ C₁-C₈-alkyle ou A-Y,
A représente C₁-C₈-alkylène ou C₂-C₈-alcénylène,
Y représente CO₂H, SO₃H, OPO(OH)₂, PO(OH)₂ ou tétrazolyle, CHO ou OH,
R⁴, R⁵, R⁶ et R⁷, qui peuvent être identiques ou différents, représentent H ou C₁-C₈-alkyle,
X représente O, S, SO, SO₂ ou NR¹⁰, où R¹⁰ représente H, C₁-C₈-alkyle ou phényle,
B représente CR¹¹R¹², où R¹¹ et R¹², qui peuvent être identiques ou différents, représentent H ou C₁-C₈-alkyle et
a représente 0, 1 ou 2, et
leurs isomères optiques, leurs sels et leurs C₁-C₈-alkyl-, pivaloyloxyméthyl-, acétoxyméthyl-, phtalidyl-, indanyl- et méthoxyméthylester, à l'exception d'un composé de formule où R¹ représente H, R² et R³ représentent phényle, a représente 0, X représente NC₂H₅ et R⁴, R⁵, R⁶ et R⁷ représentent H.

2. Composés selon la revendication 1 de formule I, où deux des groupements R¹, R² et R³ représentent indépendamment l'un de l'autre un groupement phényle, un groupement phényle substitué par 1 à 3 atomes d'halogène ou un groupement hétérocyclique aromatique défini comme dans la revendication 1, qui peut éventuellement être substitué par un atome d'halogène et
le troisième des groupements R¹, R² et R³ représente A-Y,
A représente C₁-C₈-alkylène et
Y représente CO₂H, SO₃H, CHO ou COCO₂H.

3. Composés selon la revendication 1 de formule I, où deux des groupements R¹, R² et R³ représentent indépendamment l'un de l'autre un groupement phényle, un groupement phényle substitué par 1 ou 2 atomes d'halogène ou un groupement hétérocyclique aromatique défini comme dans la revendication 1, qui peut éventuellement être substitué par un atome d'halogène,
le troisième des groupements R¹, R² et R³ représente un atome d'hydrogène,
R⁴, R⁵, R⁶ et R⁷ possèdent les significations indiquées dans la revendication 1,
X représente S, SO ou SO₂ ou NR¹⁰, où R¹⁰ représente H, C₁-C₈-alkyle ou phényle,
B représente CH₂ et
a représente 0, 1 ou 2.

4. Composés selon la revendication 2 de formule I où R¹ représente phényle et R² représente phényle ou phényle halogéno-substitué.

5. Composés selon la revendication 1 de formule I, où un ou deux des groupements R¹, R² et R³ représentent un groupement hétérocyclique aromatique éventuellement substitué défini comme dans la revendication 1.

6. Composés selon l'une des revendications précédentes de formule 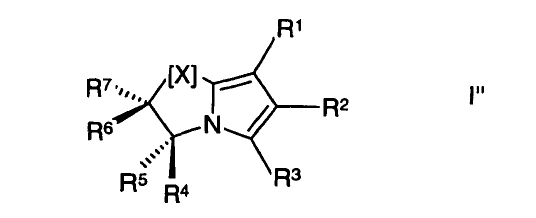 où X, R¹, R², R³, R⁴, R⁵, R⁶ et R⁷ possèdent les significations indiquées dans l'une des revendications précédentes.

7. Composés selon la revendication 6 de formule I", où X représente S, SO ou SO₂, R¹ représente phényle, R² représente 4-chlorophényle et R³ possède l'une des significations citées ci-dessus, de préférence A-Y, comme en particulier -CH₂COOH, et les groupements R⁴, R⁵, R⁶ et R⁷ représentent H ou méthyle.

8. Agent pharmaceutique contenant au moins un composé de formule I où l'un des groupements R¹, R² et R³ représente A-Y, où Y représente CO₂H ou SO₃H et les deux autres des groupements R¹, R² et R³, A, R⁴, R⁵, R⁶, R⁷, X, B et a possèdent les significations indiquées dans la revendication 1, ou un isomère optique, un sel pharmaceutiquement acceptable ou un C₁-C₈-alkyl-, pivaloyloxyméthyl-, acétoxyméthyl-, phtalidyl-, indanyl- et méthoxyméthylester de celui-ci, éventuellement en combinaison avec des supports et/ou additifs pharmaceutiquement acceptables.

9. Utilisation d'au moins un composé de formule I, défini comme dans la revendication 8, pour la production d'un agent pharmaceutique pour la prévention des maladies induites par des allergies ou pour le traitement des maladies du domaine rhumatismal.

10. Procédé de production des composés selon l'une des revendications 1 à 7, **caractérisé en ce que** l'on convertit un composé de formule générale II : 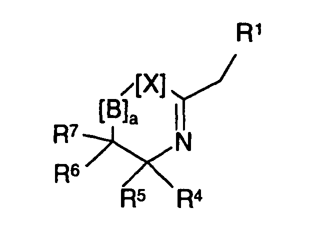 avec un composé de forme générale III : 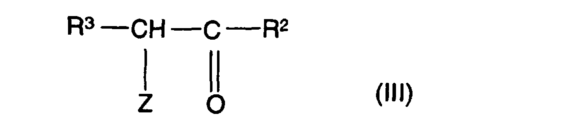 où, dans les formules ci-dessus, deux des groupements R¹, R² et R³ possèdent les significations indiquées dans la revendication 1 et le troisième représente un atome d'hydrogène et Z représente Cl ou Br, dans un éther ou un hydrocarbure aromatique, en un produit de quaternisation formé de manière intermédiaire, et on traite le produit de quaternisation dans un solvant inerte avec la triéthylamine, où on obtient un composé de formule générale la 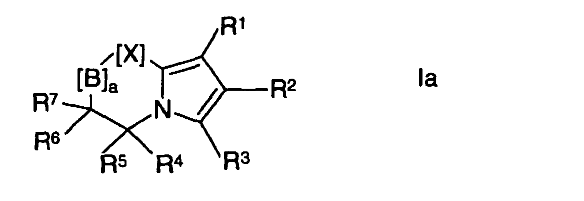 où R¹ à R⁷, B, a et X possèdent les significations indiquées ci-dessus.

11. Procédé selon la revendication 10, **caractérisé en ce que**, pour la production des composés de formule I, où le troisième des groupements R¹, R² et R³ représente CH₂COOH, CH₂COOC₁-C₈-alkyle ou COCO₂H, on convertit un composé de formule Ia définie dans la revendication 10
a) avec le chlorure d'oxalyle en un composé de formule I où l'un des groupements R¹, R² et R³ représente COCO₂H, et, si on le souhaite, on traite ce composé avec un réactif qui convient pour la réduction du groupe céto de l'acide cétocarboxylique en un groupe CH₂, de sorte que l'on obtient un composé de formule I où l'un des groupements R¹, R² et R³ représente CH₂CO₂H,
b) avec un alkylester d'acide diazoacétique en un composé de formule I où l'un des groupements R¹, R² et R³ représente CH₂COOC₁-C₈-alkyle, et, si on le souhaite, on soumet le composé obtenu à un clivage d'ester pour obtenir un composé de formule I où l'un des groupements R¹, R² et R³ représente CH₂CO₂H, ou
c) avec le chloral en un composé de formule I où l'un des groupements R¹, R² et R³ représente -CH(OH)CCl₃, et on convertit le composé obtenu en un dérivé activé et on réduit celui-ci avec le dithionite en un composé de formule I
où l'un des groupements R¹, R² et R³ représente CH₂COOH.

12. Procédé de production des composés selon la revendication 1, où X représente O ou S, **caractérisé en ce que** l'on convertit un composé de formule VI : 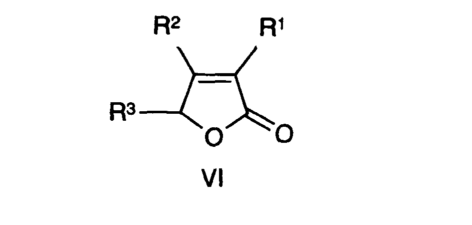 où R¹, R² et R³ possèdent les significations indiquées dans la revendication 1 avec un composé de formule 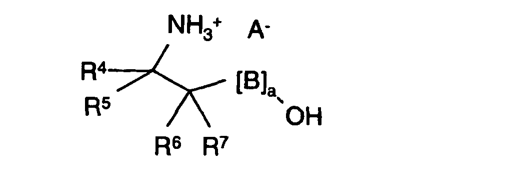 où A⁻ représente un anion, B, a et R⁴ à R⁷ possèdent les significations citées dans la revendication 1, en un composé de formule VII : 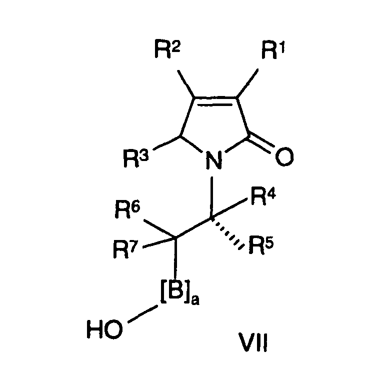 et on cyclise le composé de formule VII en un composé de formule 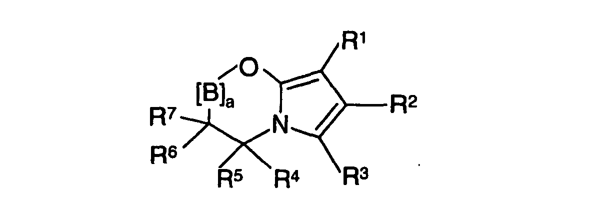 de préférence à l'aide d'acide polyphosphorique,
ou
on cyclise un composé de formule VII en présence de pentasulfure de phosphore en un composé de formule
